# FASCICULE DE BREVET EUROPEEN

(11) **EP 0 354 078 B1**
(45) Date de publication et mention de la délivrance du brevet: **10.03.1993**
(21) Numéro de dépôt: 89401896.9
(22) Date de dépôt: 03.07.1989
(51) Int. Cl.: C07C 211/42, C07C 233/14, C07C 229/14, C07C 211/30, C07C 215/42, C07C 233/36, C07C 219/14, C07D 295/06, C07D 233/64, A61K 31/135, A61K 31/165

(54) **Nouveaux dérivés du benzocycloheptène, leur procédé de préparation et les compositions pharmaceutiques les renfermant**
Derivate von Benzocyclohepten, Verfahren zu ihrer Herstellung und sie enthaltende Arzneimittel
Derivatives of benzocycloheptene, method for their preparation and pharmaceutical compositions containing same

(30) Priorité: 04.07.1988 FR 8808989
(43) Date de publication de la demande: 07.02.1990
(73) Titulaire: ADIR ET COMPAGNIE, 92415 Courbevoie Cédex (FR)
(72) Inventeur: Wierzbicki, Michel, F-78620 L'Etang la Ville (FR); Hugon, Pierre, F-92500 Rueil Malmaison (FR); Duhault, Jacques, F-78290 Croissy S/Seine (FR); Lacour, Françoise, F-94300 Vincennes (FR)
(74) Mandataire: Reverbori, Marcelle

(56) Documents cités:
- FR-A- 2 100 935
- FR-A- 2 359 608
- GB-A- 2 012 261
- US-A- 4 237 165

## Description

La présente invention a pour objet de nouveaux dérivés du benzocycloheptène, leur procédé de préparation et les compositions pharmaceutiques les renfermant.

Elle concerne particulièrement les dérivés du benzocycloheptène de formule générale I : 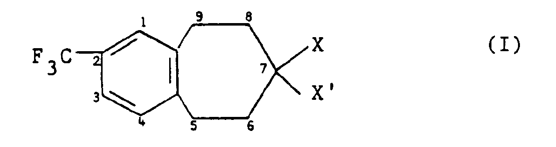 dans laquelle :
1) X représente un atome d'hydrogène, un radical alcoyle ayant de 1 à 5 atomes de carbone en chaîne droite ou ramifiée, un radical cyano ou un radical hydroxy,
2) X′ représente :
   a) un radical hydroxy,
   b) un radical OR
      dans lequel R représente :
      α)un radical acyle -COR′ dans lequel R′ représente
         un radical alcoyle ayant de 1 à 5 atomes de carbone en chaîne droite ou ramifiée éventuellement substitué par un radical phényle, ou
         un radical phényle éventuellement substitué par un ou plusieurs atomes d'halogène ou radicaux alcoyle ou alcoxy ayant chacun de 1 à 5 atomes de carbone ; ou
      β)un radical alcoyle ayant de 1 à 5 atomes de carbone en chaîne droite ou ramifiée éventuellement substitué par un radical carboxy ou un radical phényle.
   c) un radical de formule : 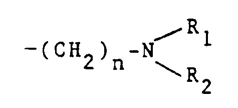
   dans laquelle :
   n représente 0, 1 ou 2, et
   R₁ et R₂, identiques ou différents, représentent chacun :
   un atome d'hydrogène ;
   un radical alcoyle ayant de 1 à 5 atomes de carbone en chaîne droite ou ramifiée éventuellement substitué par un radical hydroxy, amino, carboxy, phényle, alcoxycarbonyle ayant de 1 à 6 atomes de carbone ou acyloxy de formule R′ CO-O- dans laquelle R′ a la signification précédemment définie ;
   un radical alcényle ayant de 2 à 5 atomes de carbone ;
   un radical alcynyle ayant de 2 à 5 atomes de carbone ;
   un radical acyle de formule R˝CO dans laquelle R˝ représente un radical alcoyle ayant de 1 à 5 atomes de carbone en chaîne droite ou ramifiée,un radical phényle ou un radical phénylsulfonylamino dont la partie phényle est éventuellement substituée par un ou plusieurs atomes d'halogène ou radicaux trifluorométhyle, alcoyle ou alcoxy ayant chacun de 1 à 5 atomes de carbone, ou,
   R₁ et R₂ représentent ensemble avec l'atome d'azote auquel ils sont liés :
   - soit un groupe de formule 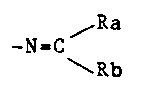 dans laquelle :
      Ra représente un atome d'hydrogène ou un radical alcoyle ayant de 1 à 5 atomes de carbone en chaîne droite ou ramifiée, et
      Rb représente un atome d'hydrogène, un radical alcoyle ayant de 1 à 5 atomes de carbone en chaîne droite ou ramifiée, un radical phényle ou un radical phénylalcoyle;
   - soit un radical hétérocyclique penta- ou hexagonal renfermant éventuellement un autre hétéroatome : oxygène ou azote ;
   ou
3) X et X′ représentent ensemble un radical de formule :

   =N-R₃

   dans laquelle
   R₃ représente un radical alcoyle ayant de 1 à 5 atomes de carbone en chaîne droite ou ramifiée éventuellement substitué par
   un radical cyano,
   un reste - COOR‴ dans lequel R‴ représente un atome d'hydrogène ou un radical alcoyle ayant de 1 à 5 atomes de carbone ; ou
   un radical phényle, lui-même éventuellement substitué par un ou plusieurs atomes d'halogène ou radicaux alcoyle ou alcoxy ayant chacun de 1 à 5 atomes de carbone ;
   sous forme racémique et les énantiomères correspondants.

L'état antérieur de la technique le plus proche de la présente invention est illustré notamment
par C.A. (1961) 21066d qui cite le 7-aminobenzocycloheptène, lequel produit fut testé comme antidépresseur, cf J. Pharm. Pharmacol. (1965), 17, 243 ;
par le brevet français 2.359.608 lequel concerne, entre autres, les amino-7 benzocycloheptènes de formule générale 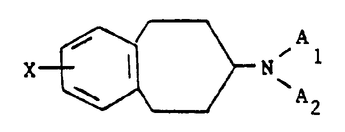
dans laquelle :
- A₁ est hydrogène, (C₁-C₅) alcoyle ou (C₂-C₅) alcényle A₂ est (C₁-C₅) alcoyle, ou (C₂-C₅) alcényle ou
- A₁ et A₂ forment ensemble avec l'atome d'azote un hétérocycle saturé ayant de 4 à 6 atomes de carbone et éventuellement un autre hétéroatome ; et
- X est hydrogène ou halogène mais n'est jamais un radical trifluorométhyle ; ces dits dérivés sont décrits comme agents anti dépresseurs ; et
   par le brevet U.S. 4.185.118 qui revendique les dérivés de formule générale :

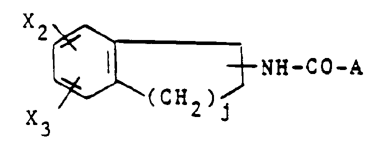

dans laquelle j est un nombre entier de 1 à 4, A est un reste d'acide gras à longue chaîne non saturée diminué de la partie carboxylique, X₂ et R₃ sont des substituants classiques mais ne représentent jamais un radical trifluorométhyle ;
ces dérivés sont décrits comme agents anti athérosclérotiques.

Il convient de remarquer que bien qu'inclus dans la formule générale, aucun dérivé de benzocycloheptène n'est exemplifié dans ce brevet ; seules les préparations des dérivés indanyle y sont décrites.

L'introduction d'un substituant trifluorométhyle en position 2 du noyau benzénique des benzocycloheptènes, antérieurement connus, a conduit à l'obtention des dérivés de formule générale I, objet de la présente invention, et a permis de mettre en évidence pour ces dérivés (I) une remarquable activité dans le traitement des maladies métaboliques, nullement mentionnée pour les composés proches antérieurement connus.

Les dérivés, objet de la présente demande, se différencient donc de ceux de l'état antérieur de la technique, à la fois par leur structure et par leur propriété, ce qui assure doublement l'originalité de la présente invention.

"En ce qui concerne l'état antérieur de la technique basé sur l'application thérapeutique, on peut citer, comme produit utilisable dans le traitement des maladies métaboliques, le benfluorex (DCI) (cf Index Nominum 1990-91), commercialisé sous le nom Mediator® (cf Dictionnaire Vidal, 1991) qui avait fait l'objet (à titre de médicament utile dans le traitement des troubles du métabolisme des carbohydrates) du brevet US 4.237.165. Les dérivés de la présente invention, qui sont structurellement bien différenciés du benfluorex, offrent, par rapport à ce dernier, une alternative intéressante dans le traitement des maladies métaboliques."

La présente invention a également pour objet le procédé de préparation des dérivés de formule générale I, caractérisé en ce que l'on fait réagir :
la trifluorométhyl-2 tétrahydro-6,7,8,9 [5H] benzocycloheptène-one-7 de formule II : 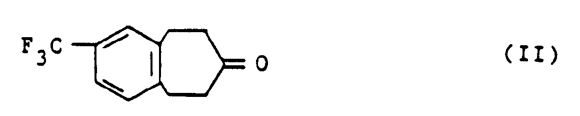
a) soit avec un dérivé carbonylé de formule générale III :

   R′₁-CO-NH-R₁ (III)

   dans laquelle R₁ a la signification énoncée précédemment et R′₁ représente :
   un atome d'hydrogène ;
   un radical alcoyle ayant de 1 à 4 atomes de carbone en chaîne droite ou ramifiée, en présence d'acide formique, pour obtenir un dérivé de formule générale Ia : 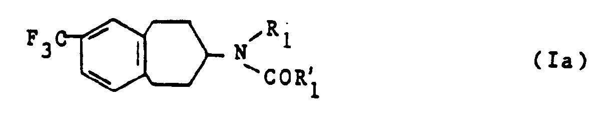 dans laquelle R₁ et R′₁ ont les significations précédemment définies,
   lequel dérivé (Ia) peut être :
   soit réduit pour donner le dérivé de formule générale Ib: 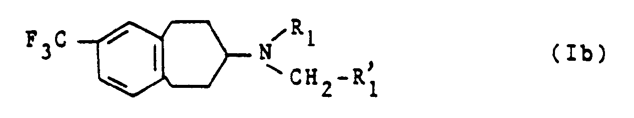 dans laquelle R₁ et R′₁ sont tels que précédemment définis,
   soit hydrolysé pour donner le dérivé de formule générale Ic : 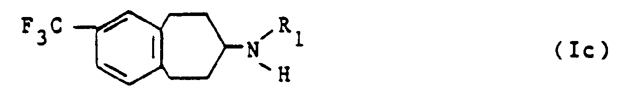 dans laquelle R₁ a la signification précédemment définie;
(b) soit avec le chlorhydrate d'hydroxylamine pour obtenir l'oxime de formule : 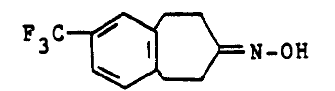 que l'on hydrogène sous pression , en présence d'un catalyseur comme le nickel de Raney, en milieu éthanol ammoniacal, pour obtenir le dérivé de formule Id : 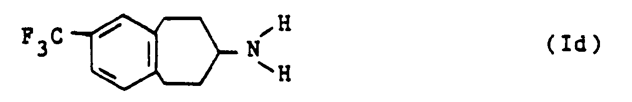 Ce dérivé (Id) peut être, à son tour, transformé selon les méthodes classiques de N-substitution, pour donner les amines secondaires et tertiaires correspondantes incluses dans la formule générale I;
c) soit avec le dérivé de formule IV

   R₁-NH₂ (IV)

   dans laquelle R₁ a la signification précédemment définie, en milieu acide chlorhydrique en présence d'un cyanure alcalin,
   pour obtenir le dérivé de formule : 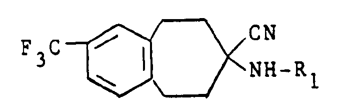 lequel est transformé en dérivé de formule : 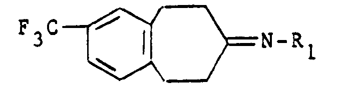 qui est, à son tour, transformé en dérivé de formule (Ie) 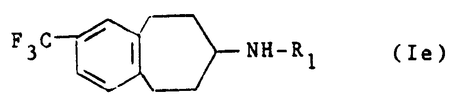 R₁ ayant, dans les trois dernières formules, la signification antérieurement définie;
d) soit avec un dérivé de formule V :

   R₄-Hal (V)

   dans laquelle R₄ représente un radical alcoyle ayant de 1 à 5 atomes de carbone en chaîne droite ou ramifiée, en présence de magnésium pour obtenir le dérivé de formule 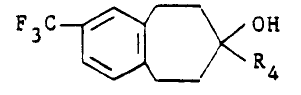 (dans laquelle R₄ a la signification précédemment définie) qui traité par un dérivé de formule R₅-CN (dans laquelle R₅ représente un radical alcoyle ayant de 1 à 4 atomes de carbone en chaîne droite ou ramifiée) en milieu sulfurique, donne le dérivé de formule : 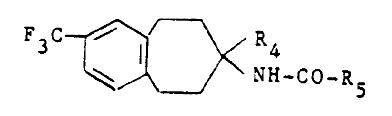 (dans laqquelle R₄ et R₅ ont les significations précédemment définies)
   lequel est réduit en un dérivé de formule If : 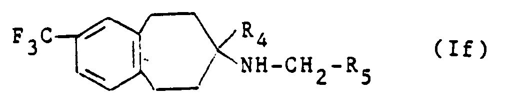 dans laquelle R₄ et R₅ ont les significations précédemment définies;
e) soit avec un dérivé de formule VI :

   MCN (VI)

   dans laquelle M représente un métal alcalin, en présence de CH₃CO OH et (CH₃CO)₂O,
   pour donner le dérivé de formule : 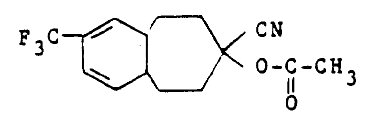 lequel est à son tour :
   soit traité par Li AlH₄ pour donner le dérivé de formule Ig : 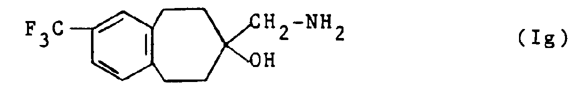 soit traité par une base en milieu aqueux pour donner le dérivé de formule : 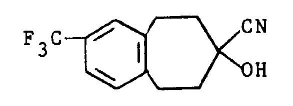 qui après déshydratation donne le dérivé de formule : 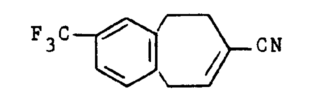 lequel par réduction donne le dérivé de formule Ih : 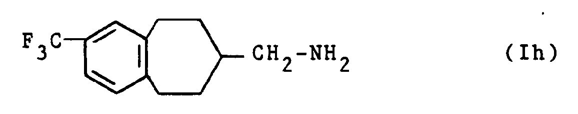
f) soit avec un dérivé de formule VII :

   PO(OC₂H₅)₂-CH₂-CN (VII)

   en présence d'une base pour donner le dérivé de formule ; 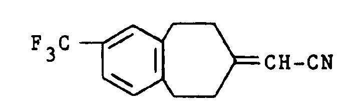 lequel par réduction donne le dérivé de formule Ii :

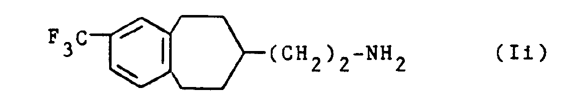

L'ensemble des dérivés de formule Ia, Ib, Ic, Id, Ie, If, Ig, Ih, et Ii forment l'ensemble des dérivés de formule générale I.

Si on le désire les dérivés I obtenus sous forme racémique peuvent être dédoublés, selon les méthodes classiques, pour obtenir les énantiomères correspondants.

En effet, les bases libres des composés de type Ib, Ic et Id dans lesquels l'atome d'azote n'est pas acylé, donnent avec un acide optiquement actif, un mélange de sels diastéréoisomères.
Ce mélange de sels peut être recristallisé. En amorçant la cristallisation avec un échantillon de sel de l'un des énantiomères purs, on précipite sélectivement le sel diastéréoisomère correspondant à cet énantiomère. La préparation de tels énantiomères purs est illustrée par les exemples 32 à 37.

La matière première dénommée trifluorométhyl-2 tétrahydro-6,7,8,9 [5H] benzocycloheptène-one-7, de formule II, est un produit nouveau, qui fait à ce titre partie de la présente invention. Il trouve son application comme matière première dans l'industrie chimique et pharmaceutique et notamment dans la synthèse des dérivés du trifluorométhyl-2 tétrahydro-6,7,8,9 [5H] benzocycloheptène de formule générale I, eux-mêmes utilisés comme médicament.

Ce dérivé de formule II a été obtenu à partir d'un produit commercial le p.trifluorométhyl iodobenzène, selon le mode opératoire ci-après exemplifié illustrant le schéma réactionnel suivant :

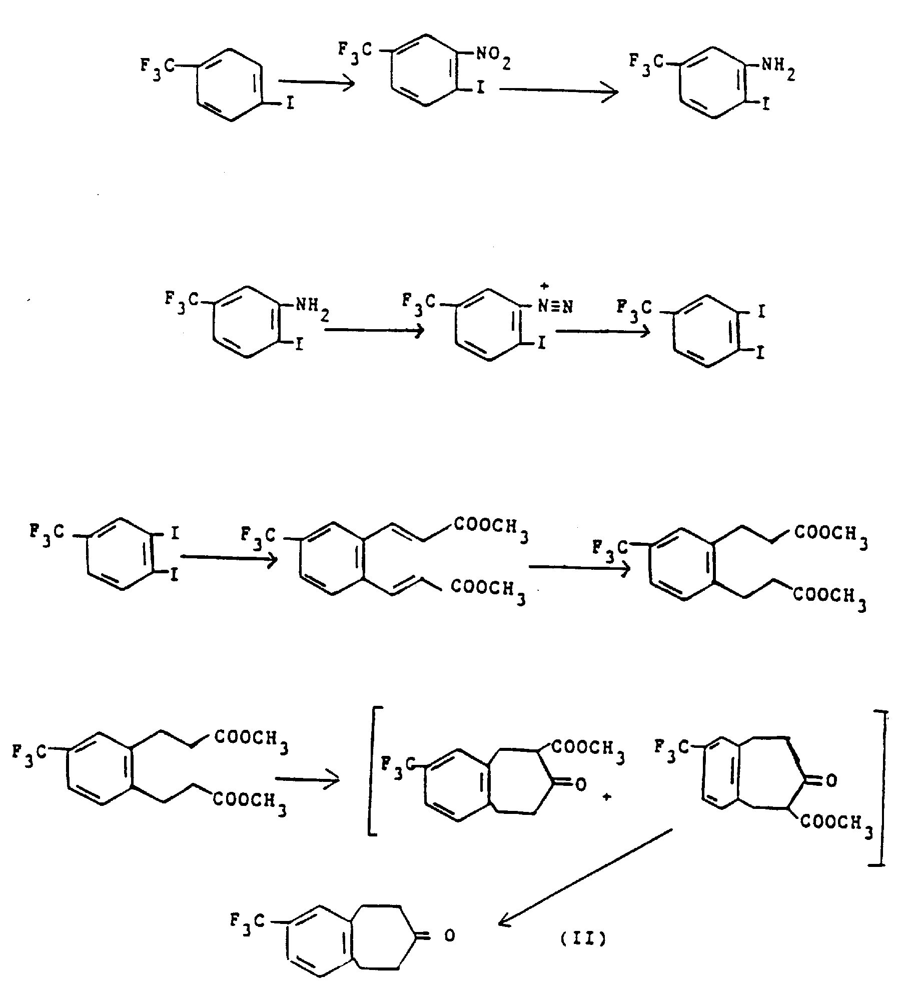

Les dérivés I renfermant un groupe basique peuvent être transformés en sels d'addition avec les acides, sels qui font, à ce titre, partie de la présente invention. Comme acides utilisables pour la formation de ces sels on peut citer, dans la série minérale : les acides chlorhydrique, bromhydrique, sulfurique, phosphorique, et dans la série organique : les acides acétique, propionique, maléique, fumarique, tartrique, citrique, benzoique et méthane sulfonique.

Les dérivés de formule générale I et leurs sels physiologiquement tolérables possèdent des propriétés pharmacologiques et thérapeutiques intéressantes notamment des propriétés régulatrices du métabolisme des glucides et des lipides qui, de ce fait permettent leur utilisation comme médicament en particulier dans le traitement des maladies métaboliques notamment les diabètes, les obésités et leurs complications : dyslipémies, hypertension artérielle, athérome etc..., et dans le traitement d'autres désordres neuro-endocrinologiques quelle que soit leur origine (diététique, génétique, traumatique ou autre).

Ces dérivés I peuvent également être avantageusement prescrits un association avec d'autres traitements utilisés dans les pathologies ci-dessus mentionnées.

En administration chronique chez des rats obèses présentant une intolérance au glucose et une hyperinsulinémie ou un diabète patent, (eu composés normalisent l'insulinémie et la tolérance au glucose dans la test de tolérance au glucose par voie veineuse (selon V. CONARD mesure de l'assimilation du glucose - bases théoriques et applications cliniques - Edition Acta Medica Belgica - Bruxelles 1955), à des doses de 0,1 à 10mg/kg, 1 à 3 fois par jour.

La présente invention a également pour objet les compositions pharmaceutiques contenant comme principe actif un composé de formule générale I ou un de ses sels physiologiquement tolérable, mélangé ou associé à un excipient pharmaceutique approprié, comme par exemple, l'eau distillée, l'amidon, le talc, l'éthylcellulose ou le stéarate de magnésium.

Les compositions pharmaceutiques ainsi obtenues se présentent généralement sous forme dosée et peuvent contenir de 10 à 100mg de principe actif. Elles peuvent revêtir, par exemple, la forme de comprimés, dragées, gélules, suppositoires, solutions injectables ou buvables, et être selon les cas admninistrées par voie orale, rectale ou parentérale à la dose de 10 à 100mg, 1 à 3 fois par jour.

Les exemples suivants illustrent l'invention. Les points de fusion sont déterminés à la platine chauffante de Kofler.

### Exemple 1 :

### Trifluorométhyl-2 tétrahydro-6,7,8,9 [5H] benzocycloheptène-one-7 :

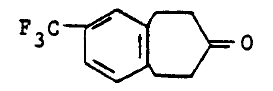

a) préparation de nitro-2 trifluorométhyl-4 iodobenzène :
   On prépare à 0°C un mélange de 87,6ml d'acide sulfurique concentré et de 46,8ml d'acide nitrique concentré (d=1,52). On additionne ce mélange goutte à goutte à 272g de p.trifluorométhyl iodobenzène maintenu à 50°C ; ce qui dure environ 1 heure ; après quoi le chauffage est arrêté et l'agitation est maintenue pendant encore 30 minutes. Le mélange est ensuite hydrolysé en le versant sur 750ml d'eau. Puis on extrait le produit avec 3 fois 600ml de dichlorométhane, et lave la phase organique avec successivement de l'eau et une solution à 20% de bicarbonate de sodium. Le solvant est ensuite évaporé et après distillation on obtient 273g de nitro-2 trifluorométhyl-4 iodobenzène Eb_{/ 1066} _{Pa} = 120-125°C (Rendement 86 %)
b) préparation d'amino-2 trifluorométhyl-4 iodobenzène :
   On porte à reflux un mélange de 225ml d'eau, 166g de fer et 10,03g de chlorure d'ammonium. On y ajoute, en 2 heures, 317g de nitro-2 trifluorométhyl-4 iodobenzène, puis on maintient le reflux pendant 3 heures 30 après la fin de l'addition. On laisse refroidir puis extrait le milieu au benzène avec 4 fois 250ml de benzène. Le mélange est ensuite filtré puis décanté. La couche organique est séchée et le solvant est évaporé. Après distillation, on recueille 256g d'amino-2 trifluorométhyl-4 iodobenzène, Eb:102à114°C, ind (25°C) : 1,556. (Rendement : 89,5%).
c) trifluorométhyl-4 di-iodo-1,2 benzène :
   On porte à 55°C un mélange de 287g d'amino-2 trifluorométhyl-4 iodobenzène et 417ml d'HCl concentré. On refroidit le mélange à une température maintenue entre -10 et -15°C et on y ajoute une solution de 38g de nitrite de sodium dans 70ml d'eau en maintenant la température entre -10 et -15°C. On ajoute alors 158ml d'HCl concentré puis une autre quantité de 38g de nitrite de sodium dans 70ml d'eau. On maintient ensuite sous agitation à une température inférieure ou égale à -10°C, pendant une heure. On prépare une solution de 250g d'iodure de potassium dans 400ml d'eau ; on refroidit à 0°C et y ajoute 54,3ml d'H₂SO₄ concentré en refroidissant. On ajoute à ce mélange, la solution de dérivé diazoique précédemment obtenue en maintenant la température inférieure à 5°C. Une fois l'addition terminée, on maintient l'ensemble à température ambiante pendant 15 minutes, puis le porte à reflux pendant une demie heure et enfin le laisse refroidir. On extrait le mélange avec 3 fois 1600ml d'éther, lave l'extrait avec une solution à 12,5% de bisulfite de sodium puis à l'eau, sèche, puis distille l'éther. On obtient 377g de trifluorométhyl-4 di-iodo-1,2 benzène, P.F. : 62-64°C. (Rendement : 95%).
d) préparation de trifluorométhyl-4 di (β-méthoxycarbonylvinyl)-1,2 benzène :
   Un mélange de 398g de trifluorométhyl-4 di-iodo-1,2 benzène, 224ml d'acrylate de méthyle, 24,3g de tri-o.tolyl phosphine, 4,5g d'acétate de palladium, 342ml de triéthylamine et 1450ml de xylène anhydre est maintenu, sous agitation, à une température d'environ 80°C, pendant 36 heures. Puis le chauffage est arrêté et le mélange filtré. Le résidu est lavé avec 2 fois 250ml de xylène, puis le xylène est distillé sous vide. Le résidu est lavé par un mélange de cyclohexane, éther de pétrole (25ml/25ml), repris par le minimum d'éther éthylique. La phase éthérée est lavée à l'eau, décantée, séchée, décolorée sur noir animal, puis l'éther est distillé. On obtient 236g de trifluorométhyl-4 di-(β-méthoxycarbonylvinyl)-1,2 benzène (Rendement 75%).
e) préparation de trifluorométhyl-4 di-(β-méthoxycarbonyléthyl)-1,2 benzène ;
   314g de trifluorométhyl-4 di-(β-méthoxycarbonylvinyl)-1,2 benzène dissous dans 2200ml de diméthylformamide et 1500ml de méthanol sont hydrogénés sous une pression de 5,5 à 5,6.10⁵ Pa en présence d'environ 300ml d'une suspension de nickel de Raney. Le mélange est ensuite filtré, le solvant distillé et l'huile brute pratiquement pure recueillie est utilisée telle quelle. On obtient ainsi 305g de trifluorométhyl-4 di-(β-méthoxycarbonyléthyl)-1,2 benzène, (Rendement ≃ 96%).
f) préparation du mélange de trifluorométhyl-2 méthoxycarbonyl-6 tétrahydro-6,7,8,9-[5H] benzocycloheptène-one 7, et de trifluorométhyl-2 méthoxycarbonyl-8 tétrahydro-6,7,8,9-[5H] benzocycloheptène-one-7 :
   On ajoute 44g de suspension d'hydrure de sodium (à 60% dans l'huile) à 1000ml de xylène, et on porte le tout à 90°C. On y ajoute goutte à goutte un mélange de 318g de trifluorométhyl-4 di-(β-méthoxycarbonyléthyl)-1,2 benzène et 500ml de xylène, sous agitation. On porte à reflux (après la fin du dégagement gazeux) pendant environ 4 heures. La température est ramenée à 20°C. On ajoute au mélange 1000ml d'eau et extrait la couche organique. La couche aqueuse est lavée par 3 fois 500ml d'éther. Les couches organiques sont rassemblées et les solvants distillés. Le mélange résiduel est séché et utilisé tel quel. On obtient 242g de mélange (Rendement ≃ 85%).
g)préparation de trifluorométhyl-2 tétrahydro-6,7,8,9 [5H] benzocycloheptène-one-7 :
   On porte à reflux pendant 15 minutes 285g du mélange d'esters cétoniques précédemment obtenu, 1000ml de solution normale de soude et 1000ml d'éthanol. Après quoi, l'éthanol est éliminé par distillation sous pression réduite et le mélange aqueux résiduel est extrait par 4 fois 1500ml d'éther éthylique. On sèche la couche organique, reprend par le minimum de dichlorométhane et purifie en filtrant sur environ 2000g de silice ou en recristallisant dans le cyclohexane. On obtient ainsi 168g de trifluorométhyl-2 tétrahydro-6,7,8,9 [5H] benzocycloheptène-one-7, P.F. : 78°C (Rendement 74%).

### Exemple 2 :

### Trifluorométhyl-2 amino-7 tétrahydro-6,7,8,9 [5H] benzocycloheptène :

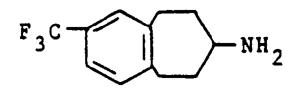

On porte à reflux pendant 3 heures un mélange de 228g de trifluorométhyl-2 tétrahydro-6,7,8,9 [5H] benzocycloheptène-one-7, 69,5g de chlorhydrate d'hydroxylamine et 550ml de pyridine. On distille la pyridine sous pression réduite, reprend le résidu par 2200ml d'eau et extrait par 4 fois 4000ml d'éther.
Les phases éthérées sont séchées, l'éther est distillé et le résidu brut obtenu (231g), P.F. 117-118°C est utilisé tel quel. (Rendement ≃ 95%). 243g de l'oxime ci-dessus obtenue sont mis en solution dans un mélange de 1200ml d'éthanol et 5000ml d'éthanol ammoniacal puis hydrogénés sous une pression de 5,5 à 5,6.10⁵ Pa, d'abord à température ambiante, puis à 40°C après 1 heure 30 et ce pendant 10 à 15 minutes. On filtre ensuite le mélange réactionnel et distille le solvant jusqu'à obtention d'une base huileuse brute. On obtient ainsi 222g de trifluorométhyl-2 amino-7 tétrahydro-6,7,8,9 [5H] benzocycloheptène (Rendement ≃ 97%). Cette base est purifiée sous forme de sel, notamment comme suit sous forme de chlorhydrate :
22,9g de base précédemment obtenue sont dissouts dans 200ml d'isopropanol. On y ajoute, sous agitation, 25ml d'éther chlorhydrique, 4N. Le précipité formé est filtré, redissout à chaud dans le minimum du mélange CH₂Cl₂/CH₃OH (80/20). On concentre le mélange progressivement jusqu'à précipitation, filtre le précipité, le lave à l'éther, le sèche et on obtient ainsi avec un rendement de 80-85% le chlorhydrate de trifluorométhyl-2 amino-7 tétrahydro-6,7,8,9 [5H] benzocycloheptène, P.F. > 260°C avec sublimation vers 300°C.

### Exemple 3

### Trifluorométhyl-2 (N-éthyl N-formyl amino)-7 tétrahydro-6,7,8,9 [5H] benzocycloheptène :

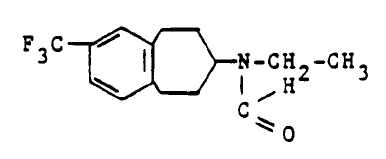

Un mélange de 228,2g de trifluorométhyl-2 tétrahydro-6,7,8,9 [5H] benzocycloheptène-one-7, 219g de N-éthylformamide et 151g d'acide formique est chauffé à reflux pendant 14 heures. Après concentration sous vide, en chauffant au bain-marie, le résidu est repris par 2300ml de chlorure de méthylène. Après lavage par 2 fois 800ml d'eau, la couche organique est séchée sur sulfate de magnésium et après filtration, le solvant est éliminé sous vide. On obtient 283g de trifluorométhyl-2 (N-éthyl N-formyl amino)-7 tétrahydro-6,7,8,9 [5H]benzocycloheptène, sous forme d'huile.

### Exemple 4 :

### Trifluorométhyl-2 N-éthylamino-7 tétrahydro-6,7,8,9 [5H] benzocycloheptène :

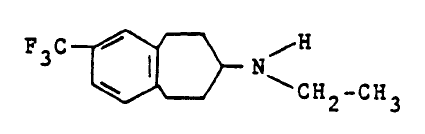

On chauffe à reflux pendant 12 heures sous agitation un mélange de 285,31g de trifluorométhyl-2 (N-éthyl N-formyl amino)-7 tétrahydro-6,7,8,9 [5H] benzocycloheptène, 1150ml d'éthanol, 270ml d'acide chlorhydrique concentré (d:1,19) et 215ml d'eau. Le mélange réactionnel est ensuite concentré sous vide, en chauffant au bain-marie.
Le résidu est trituré avec 2000ml d'éther anhydre. Le précipité est filtré, séché à l'air. On obtient 235g de produit fondant à 190-200°C.
Après recristallisation dans 400ml d'isopropanol bouillant on obtient 150g de trifluorométhyl-2 N-éthylamino-7 tétrahydro-6,7,8,9 [5H] benzocycloheptène. P.F. : 225°C.

### Exemple 5 :

### Trifluorométhyl-2 (N-éthyl N-méthylamino)-7 tétrahydro-6,7,8,9 [5H] benzocycloheptène :

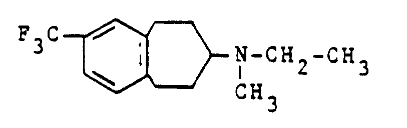

A une suspension de 56g d'hydrure de lithium-aluminium dans 4700ml d'éther anhydre, on coule, sous agitation, en une heure, une solution de 285,3g de trifluorométhyl-2 (N-éthyl N-formylamino)-7 tétrahydro-6,7,8,9 [5H] benzocycloheptène dans 3700ml d'éther anhydre (on observe un léger reflux). On chauffe ensuite à ébullition pendant 5 heures. Après refroidissement, le mélange réactionnel est hydrolysé successivement par 56ml d'eau, 56ml d'une solution 4N de soude puis par 170ml d'eau. Après filtration et lavage à l'éther, la couche organique est concentrée sous vide et le résidu distillé. On obtient 150g de trifluorométhyl-2 (N-éthyl N-méthylamino)-7 tétrahydro-6,7,8,9 [5H] benzocycloheptène, Eb/_{13,3 Pa} = 135-140°C.

### Exemple 6 :

### Trifluorométhyl-2 (N-éthoxycarbonylméthylamino)-7 tétrahydro-6,7,8,9 [5H] benzocycloheptène.

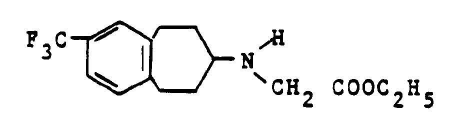

On dissout 458,5g (2moles) de trifluorométhyl-2 amino-7 tétrahydro-6,7,8,9 [5H] benzocycloheptène dans 20̸00ml de benzène anhydre. On y ajoute une mole de bromo-acétate d'éthyle et on porte à reflux. Un précipité se forme. On maintient le reflux pendant 4 heures,puis laisse revenir le mélange à la température ambiante. On filtre le précipité de bromhydrate de l'amine de départ et on lave au benzène puis à l'éther.
On distille le solvant du filtrat. L'huile obtenue est utilisée telle quelle pour la saponification ultérieure. On obtient finalement 271g de trifluorométhyl-2 (N-éthoxycarbonylméthylamino)-7 tétrahydro-6,7,8,9 [5H] benzocycloheptène. (Rendement 86%) dont un échantillon est purifié sous forme de chlorhydrate,qui se sublime vers 270-280°C.

### Exemple 7 :

### Trifluorométhyl-2 (N-carboxyméthylamino)-7 tétrahydro-6,7,8,9 [5H] benzocycloheptène.

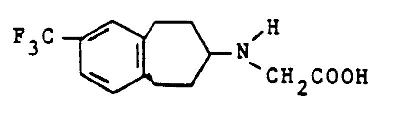

315,34g (1 mole) de trifluorométhyl-2 (N-éthoxycarbonylméthylamino)-7 tétrahydro-6,7,8,9 [5H] benzocycloheptène sont portés à reflux pendant environ 25 minutes dans un mélange de 1000ml de soude normale et 2200ml d'éthanol. On distille ensuite l'éthanol en terminant sous pression réduite et lave la phase aqueuse avec 2 fois 4000ml d'éther éthylique. Les phases éthérées sont relavées à l'eau. Aux phases aqueuses rassemblées, on additionne 1000ml d'une solution normale d'acide chlorhydrique. Le précipité obtenu est recueilli,lavé. On obtient 250g de trifluorométhyl-2 (N-carboxyméthylamino)-7 tétrahydro-6,7,8,9 [5H] benzocycloheptène qui se sublime au dessus de 260°C. (Rendement 87%). Par addition d'une quantité stoechiométrique d'acide chlorhydrique et recristallisation dans l'eau on obtient le chlorhydrate de cet amino acide qui se sublime vers 220-222°C.

### Exemple 8 :

### Trifluorométhyl-2 (N-acétylamino)-7 tétrahydro-6,7,8,9 [5H] benzocycloheptène.

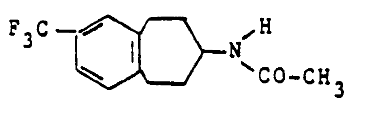

On introduit 1 mole de trifluorométhyl-2 amino-7 tétrahydro-6,7,8,9 [5H] benzocycloheptène et 1 mole de triéthylamine dans 10000ml d'éther éthylique. On ajoute lentement à ce mélange 1 mole de CH₃ CO Cl, puis on porte l'ensemble à reflux sous agitation pendant 30 minutes.
On laisse refroidir, distille l'éther sous pression réduite, reprend par l'eau et filtre le résidu obtenu. Après recristallisation dans l'acétate d'éthyle on obtient 250g de trifluorométhyl-2 N-acétylamino-7 tétrahydro-6,7,8,9 [5H] benzocycloheptène, fondant à 216-217°C avec légère sublimation. (Rendement 92%).

### Exemple 9 :

### Trifluorométhyl-2 (β-aminoéthyl)-7 tétrahydro-6,7,8,9 [5H] benzocycloheptène.

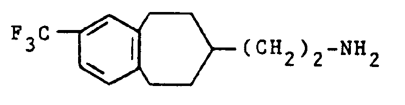

A une suspension d'hydrure de sodium (0,05 mole) (lavé à l'éther de pétrole) dans 75ml de diméthylformamide anhydre, sont ajoutés, sous agitation, 8,85g de cyanométhylènephosphonate d'éthyle, en 30 minutes. La température est ensuite portée à 50°C jusqu'à fin du dégagement d'hydrogène. Après retour à température ambiante 11,4g de trifluorométhyl-2 benzocycloheptènone-7 en solution dans 25ml de diméthylformamide sont ajoutés en 10 minutes. La température s'élève jusqu'à 43°C. Le mélange réactionnel est ensuite chauffé à 80°C pendant 1 heure 30. Après refroidissement, on reprend par 50ml d'eau et 150ml d'éther. On agite, décante, sèche la couche organique sur MgSO₄ et évapore le solvant. On obtient 12,5g de trifluorométhyl-2 chlorométhylène-7 benzocycloheptène fondant à 70°C.
6g du produit ainsi obtenu sont hydrogénés en solution dans 150ml de diméthylformamide anhydre en présence de 2,5g de palladium sur noir à 5%, sous 60 lb/s inch.
Après la fin de l'absorption, le catalyseur est essoré et le filtrat tiré à sec sous vide.
On obtient 6g de trifluorométhyl-2 cyanométhyl-7 benzocycloheptène.
Le produit précédemment obtenu en solution dans 20ml d'éther anhydre, est coulé, sous agitation, sur une suspension de 1g d'hydrure de lithium-aluminium dans 100ml d'éther anhydre en 30 minutes, à une température de -10.15°C. Après une heure d'agitation à température ambiante on hydrolyse par de la soude diluée, essore, et sèche le filtrat sur MgSO₄. Après évaporation du solvant, l'huile résultante (5,2g) est dissoute dans 25ml d'éther anhydre et après addition de 7ml d'éther chlorhydrique 3N le précipité formé est essoré et séché sous vide donnant ainsi 2,8g de chlorhydrate de trifluorométhyl-2 (β-aminoéthyl)-7 tétrahydro-6,7,8,9 [5H] benzocycloheptène, P.F.: 180-181°C.

### Exemple 10 :

### Trifluorométhyl-2 n.butylamino-7 tétrahydro-6,7,8;9 [5H] benzocycloheptène :

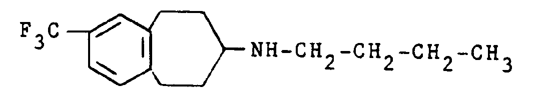

On ajoute en 5 minutes 1,95g de cyanure de potassium en solution dans 7,5ml d'eau, à une suspension de 6,8g de trifluorométhyl-2 benzocycloheptènone-7,3,25g de chlorhydrate de n.butylamine dans 6ml de méthanol. La suspension se fluidifie et la température s'élève à 29°C.
Le mélange réactionnel est agité 24 heures à température ambiante puis chauffé 3 heures à reflux.
Après refroidissement, on le reprend par 50ml de chlorure de méthylène, décante, lave par 2 fois 25ml d'eau, sèche sur MgSO₄ et concentre sous vide. On obtient 9,5g de trifluorométhyl-2 n.butylamino-7 cyano-7 benzocycloheptène. Le produit ainsi obtenu est dissous dans 200ml de benzène anhydre. On ajoute 6,7g de tertiobutylate de potassium et maintient à reflux sous agitation pendant 3 heures. Après refroidissement, la suspension est filtrée et le filtrat est concentré sous vide.
On obtient 6,5g de trifluorométhyl-2 n.butylimino-7 benzocycloheptène.
Le produit ainsi obtenu est dissous dans 150ml de méthanol. On l'hydrogène en présence de 0,7g d'oxyde de platine dans une fiole de PARR sous 4,1.10⁵ Pa (60 lb/s inch).
Après 1 heure d'agitation, l'absorption étant terminée, le catalyseur est essoré et le filtrat tiré à sec sous vide. L'huile restant (6g) est reprise par 50ml d'isopropanol anhydre et après addition de 4,3ml d'éther chlorhydrique 3N, la solution est laissée au repos. Après 24 heures à température ambiante le précipité formé est essoré et séché. On obtient 3,8g de chlorhydrate de trifluorométhyl-2 n.butylamino-7 tétrahydro-6,7,8,9 [5H] benzocycloheptène, P.F.: 234°C.

### EXEMPLE 11 :

### Trifluorométhyl-2 hydroxy-7 aminométhyl-7 tétrahydro-6,7, 8,9 [5H] benzocycloheptène :

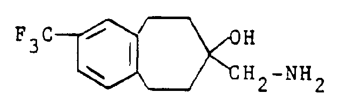

A une suspension agitée de trifluorométhyl-2 benzocycloheptènone-7 dans 5,6ml d'anhydride acétique, est ajoutée à 10°C, en 30 minutes, une solution de 3,9g de cyanure de potassium dissous dans 8ml d'eau. La température s'élève jusqu'à 25°C. Après 4 heures d'agitation à température ambiante, on verse le mélange réactionnel sur 50ml de bicarbonate de sodium à 10% dans l'eau, extrait à l'éther, lave à l'eau, sèche et concentre. On obtient 7,5g d'un produit solide, fondant à 110-113°C.
Ce solide est repris par 16ml d'anhydride acétique et 0,8ml de chlorure d'acétyle.
On agite, laisse reposer 24 heures, neutralise avec 250ml de bicarbonate de sodium à 10%, extrait à l'éther, lave à l'eau, sèche et évapore le solvant. On obtient 8,8g de trifluorométhyl-2 acétoxy-7 cyano-7 benzocycloheptène (huile) Le produit ainsi obtenu, mis en solution dans 30ml d'éther anhydre est coulé sous agitation dans une suspension de 2,5g d'hydrure de lithium aluminium.
Après 4 heures de reflux, le milieu réactionnel est hydrolysé.
Après traitement, on obtient 7,2g d'huile qui reprise par 50ml d'acétate d'éthyle anhydre et traitée par 10ml d'éther chlorhydrique 3N, donne après essorage et séchage du précipité 6,7g de chlorhydrate de trifluorométhyl-2 hydroxy-7 aminométhyl-7 tétrahydro-6,7,8,9 [5H] benzocycloheptène, P.F.: 217°C.

### EXEMPLE 12 :

### Trifluorométhyl-2 méthyl-7 éthylamino-7 tétrahydro-6,7,8,9 [5H] benzocycloheptène :

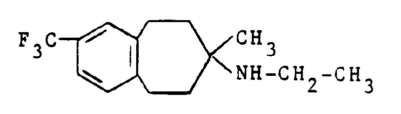

11,4g de trifluorométhyl-2 benzocycloheptènone-7 en solution dans 90ml de tétrahydrofuranne anhydre sont ajoutés, en 30 minutes, sous agitation, à une solution d'iodure de méthylmagnésium préparée à partir de 9,2g d'iodure de méthyle et 1,6g de magnésium en tournures dans 90ml d'éther anhydre. Après 3 heures de reflux on hydrolyse par 25ml d'eau, décante la couche aqueuse, sèche la couche organique sur MgSO₄ et évapore le solvant sous vide. On obtient 13g de trifluorométhyl-2 hydroxy-7 méthyl-7 benzocycloheptène.
A 10g d'acide sulfurique concentré sont ajoutés 40ml d'acide acétique glacial à température inférieure à 15°C, puis 2,2g d'acétonitrile et 13g du produit précédemment obtenu en solution dans 10ml d'acide acétique sont additionnés en 10 minutes à une température inférieure à 15°C. Après retour à température ambiante le mélange réactionnel est chauffé à 50-55°C pendant 2 heures. On le traite ensuite, après refroidissement, par 200g d'un mélange d'eau et glace et extrait à l'éther. Après séchage et évaporation du solvant sous vide, on obtient 8,7g de trifluorométhyl-2 méthyl-7 acétylamino-7 benzocycloheptène.
Ce produit dissout dans 85ml de tétrahydrofuranne anhydre est coulé, sous agitation, dans une suspension de 1,5g d'hydrure de lithium aluminium dans 120ml d'éther anhydre. Après 7 heures de reflux, hydrolyse, et essorage, le filtrat est séché sur MgSO₄ et tiré à sec sous vide. On obtient 4,8g d'une huile qui reprise par 50ml d'isopropanol et traitée par 6ml d'éther chlorhydrique 4,3N donne un précipité qui essoré, lavé et séché sous vide donne 4,8g de chlorhydrate de trifluorométhyl-2 méthyl-7 éthylamino-7 tétrahydro-6,7,8,9 [5H] benzocycloheptène, P.F. > 260°C.

### Exemples 13 à 31 :

Les dérivés suivants ont été préparés selon les méthodes ci-dessus exemplifiées.
13) Trifluorométhyl-2 N-méthylamino-7 tétrahydro-6,7,8,9 [5H] benzocycloheptène.
14) Trifluorométhyl-2 N-propylamino-7 tétrahydro-6,7,8,9 [5H] benzocycloheptène, P.F. du chlorhydrate correspondant: 231°C.
15) Trifluorométhyl-2 N-propionylamino-7 tétrahydro-6,7,8,9 [5H] benzocycloheptène.
16) Trifluorométhyl-2 N-butyrylamino-7 tétrahydro-6,7,8,9 [5H] benzocycloheptène.
17) Trifluorométhyl-2 N-hydroxyéthylamino-7 tétrahydro-6,7,8,9 [5H] benzocycloheptène, P.F. du chlorhydrate correspondant : 175°C.
18) Trifluorométhyl-2 N-diéthylamino-7 tétrahydro-6,7,8,9 [5H] benzocycloheptène.
19) Trifluorométhyl-2 N-dipropylamino-7 tétrahydro-6,7,8,9 [5H] benzocycloheptène, (huile).
20) Trifluorométhyl-2 N-diméthylamino-7 tétrahydro-6,7,8,9 [5H] benzocycloheptène.
21) Trifluorométhyl-2 (N-méthyl N-propylamino)-7 tétrahydro-6,7,8,9 [5H] benzocycloheptène, P.F. du chlorhydrate correspondant : 185°C.
22) Trifluorométhyl-2 pipéridino-7 tétrahydro-6,7,8,9 [5H] benzocycloheptène.
23) Trifluorométhyl-2 morpholino-7 tétrahydro-6,7,8,9 [5H] benzocycloheptène.
24) Trifluorométhyl-2 N-pipérazinyl-7 tétrahydro-6,7,8,9 [5H] benzocycloheptène.
25) Trifluorométhyl-2 N-imidazolidinyl-7 tétrahydro-6,7,8,9 [5H] benzocycloheptène.
26) Trifluorométhyl-2 N-propilydène amino-7 tétrahydro-6,7,8,9 [5H] benzocycloheptène.
27) Trifluorométhyl-2 N-benzoyloxyéthylamino-7 tétrahydro-6,7,8,9 [5H] benzocycloheptène, P.F. du chlorhydrate correspondant : 229-230°C avec sublimation.
28) Trifluorométhyl-2 hydroxy-7 tétrahydro-6,7,8,9 [5H] benzocycloheptène, P.F. : 80-85°C.
29) Trifluorométhyl-2 [N-éthyl N-(β-aminoéthyl) amino]-7 tétrahydro-6,7,8,9 [5H] benzocycloheptène, P.F. du dichlorhydrate correspondant > 260°C.
30) Trifluorométhyl-2 [N-éthyl N-(β-acétylaminoéthyl) amino]-7 tétrahydro-6,7,8,9 [5H] benzocycloheptène, P.F. du chlorhydrate correspondant : 167-168°C.
31) Trifluorométhyl-2 aminométhyl-7 tétrahydro-6,7,8,9 [5H] benzocycloheptène, P.F. du chlorhydrate correspondant : 198-199°C.

### Exemples 32 à 37 :

### Préparation des énantiomères :

32) Diastéréoisomère α du trifluorométhyl-2 [N-formyl N-(α-méthylbenzyl) amino]-7 tétrahydro-6,7,8,9 [5H] benzocycloheptène.
   Le trifluorométhyl-2 [N-formyl N-(α-méthylbenzyl) amino]-7 tétrahydro-6,7,8,9 [5H] benzocycloheptène a été préparé par réaction de la trifluorométhyl-2 tétrahydro-6,7,8,9 [5H] benzocycloheptène-one-7 avec le dérivé N-formylé de l'α-méthylbenzylamine, selon la mode opératoire général décrit en page 5.
   Le diastéréoisomère α est séparé du diastéréoisomère β (objet de l'exemple 33) par chromatographie sur gel de silice en utilisant comme éluant un mélange cyclohexane-acétate d'éthyle (80-20).
33) Diastéréoisomère β du trifluorométhyl-2 [N-formyl N-(α-méthylbenzyl) amino]-7 tétrahydro-6,7,8,9 [5H] benzocycloheptène, préparé selon la processus décrit dans l'exemple 32.
34) Diastéréoisomère α du trifluorométhyl-2 [N-(α-méthylbenzyl) amino]-7 tétrahydro-6,7,8,9 [5H] benzocycloheptène, obtenu par coupure du radical formyl, en milieu eau-éthanol-acide chlorhydrique, à partir du dérivé objet de l'exemple 32.
35) Diastéréoisomère β du trifluorométhyl-2 [N-(α-méthylbenzyl) amino]-7 tétrahydro-6,7,8,9 [5H] benzocycloheptène, obtenu par coupure du radical formyl, en milieu eau-éthanol-acide chlorhydrique, à partir du dérivé objet de l'exemple 33.
36) d. trifluorométhyl-2 amino-7 tétrahydro-6,7,8,9 [5H] benzocycloheptène, [dont les constantes physiques du chlorhydrate sont : P.F.>260°C (isopropanol) [α] (C=1%, CH₃OH) : 546 : + 4,9 ; 436 : + 6,3 ; 365 : + 5,7], obtenu par débenzylation catalytique (palladium/éthanol) du dérivé de l'exemple 34.
37) l. trifluorométhyl-2 amino-7 tétrahydro-6,7,8,9 [5H] benzocycloheptène, [dont les constantes physiques du chlorhydrate sont : P.F.>260°C (isopropanol) [α] (C=1%,CH₃,OH): 546 : - 4,9 ; 436 : - 6,3 ; 365 : - 5,7], obtenu par débenzylation catalytique (palladium/éthanol) du dérivé de l'exemple 35.

## Revendications (Revendications pour l'(les) Etat(s) contractant(s) suivant(s): DE, GB, FR, IT, NL, SE, LI, CH, BE, AT, LU)

1. Les dérivés du benzocycloheptène de formule générale I : 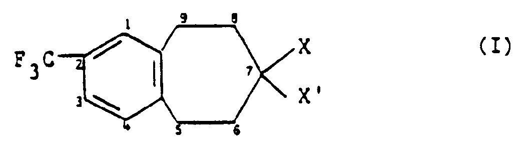 dans laquelle :
1- X représente un atome d'hydrogène, un radical alcoyle ayant de 1 à 5 atomes de carbone en chaîne droite ou ramifiée, un radical cyano ou un radical hydroxy,
2- X représente :
a) un radical hydroxy,
b) un radical OR
dans lequel R représente :
α)un radical acyle -COR′ dans lequel R′ représente
un radical alcoyle ayant de 1 à 5 atomes de carbone en chaîne droite ou ramifiée éventuellement substitué par un radical phényle, ou
un radical phényle éventuellement substitué par un ou plusieurs atomes d'halogène ou radicaux alcoyle ou alcoxy ayant chacun de 1 à 5 atomes de carbone ; ou
β)un radical alcoyle ayant de 1 à 5 atomes de carbone en chaîne droite ou ramifiée éventuellement substitué par un radical carboxy ou un radical phényle.
c) un radical de formule : 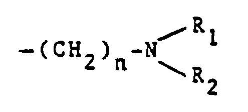
dans laquelle :
n représente 0, 1 ou 2, et
R₁ et R₂, identiques ou différents, représentent chacun :
un atome d'hydrogène ;
un radical alcoyle ayant de 1 à 5 atomes de carbone en chaîne droite ou ramifiée éventuellement substitué par un radical hydroxy, amino, carboxy, alcoxycarbonyle ayant de 1 à 6 atomes de carbone ou acyloxy de formule R′ CO-O- dans laquelle R′ a la signification précédemment définie ;
un radical alcényle ayant de 2 à 5 atomes de carbone ;
un radical alcynyle ayant de 2 à 5 atomes de carbone ;
un radical acyle de formule R˝CO dans laquelle R˝ représente un radical alcoyle ayant de 1 à 5 atomes de carbone en chaîne droite ou ramifiée, un radical phényle ou un radical phénylsulfonylamino dont la partie phényle est éventuellement substituée par un ou plusieurs atomes d'halogène ou radicaux trifluorméthyle, alcoyle ou alcoxy ayant chacun de 1 à 5 atomes de carbone.
ou,
R₁ et R₂ représentent ensemble avec l'atome d'azote auquel ils sont liés :
- soit un groupe de formule 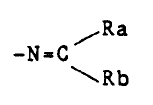 dans laquelle:
Ra représente un atome d'hydrogène ou un radical alcoyle ayant de 1 à 5 atomes de carbone en chaîne droite ou ramifiée, et
Rb représente un atome d'hydrogène, un radical alcoyle ayant de 1 à 5 atomes de carbone en chaîne droite ou ramifiée, un radical phényle ou un radical phénylalcoyle;
- soit un radical hétérocyclique penta- ou hexagonal renfermant éventuellement un autre hétéroatome : oxygène ou azote ;
ou
3- X et X′ représentent ensemble un radical de formule :
=N-R₃
dans laquelle
R₃ représente un radical alcoyle ayant 1 à 5 atomes de carbone en chaîne droite ou ramifiée éventuellement substitué par
un radical cyano,
un reste - COOR‴ dans lequel R‴ représente un atome d'hydrogène ou un radical alcoyle ayant de 1 à 5 atomes de carbone ; ou
un radical phényle, lui-même éventuellement substitué par un ou plusieurs atomes d'halogène ou radicaux alcoyle ou alcoxy ayant chacun de 1 à 5 atomes de carbone ;
sous forme racémique et les énantiomères correspondants.

2. Les dérivés de la revendication 1 répondant à la formule générale I′ : 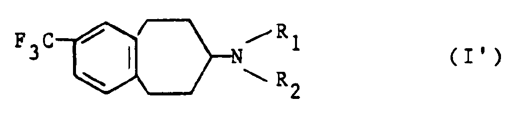 dans laquelle R₁ et R₂ ont les significations définies dans la revendication 1.

3. Les sels des dérivés de la revendication 1 renfermant un groupe basique avec des acides appropriés.

4. Les sels selon la revendication 3 qui sont physiologiquement tolérables.

5. Le trifluorométhyl-2 amino-7 tétrahydro-6,7,8,9 [5H] benzocycloheptène.

6. Le trifluorométhyl-2 (N-éthyl N-formylamino)-7 tétrahydro-6,7,8,9 [5H] benzocycloheptène.

7. Le trifluorométhyl-2 N-éthylamino-7 tétrahydro-6,7,8,9 [5H] benzocycloheptène.

8. Le trifluorométhyl-2 (N-éthyl N-méthylamino)-7 tétrahydro-6,7,8,9 [5H] benzocycloheptène.

9. Le trifluorométhyl-2 (N-éthoxycarbonylméthylamino)-7 tétrahydro-6,7,8,9 [5H] benzocycloheptène.

10. Le trifluorométhyl-2 (N-carboxyméthylamino)-7 tétrahydro-6,7,8,9 [5H] benzocycloheptène.

11. Le procédé de préparation des dérivés de la revendication 1 caractérisé en ce que l'on fait réagir :
la trifluorométhyl-2 tétrahydro-6,7,8,9 [5H] benzocycloheptène-one-7 de formule II : 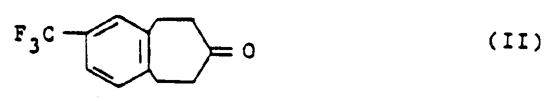
a) soit avec un dérivé carbonylé de formule générale III :
R′₁-CO-NH-R₁ (III)
dans laquelle R₁ a la signification énoncée précédemment et R′₁ représente :
un atome d'hydrogène ;
un radical alcoyle ayant de 1 à 4 atomes de carbone en chaîne droite ou ramifiée, en présence d'acide formique, pour obtenir un dérivé de formule générale Ia : 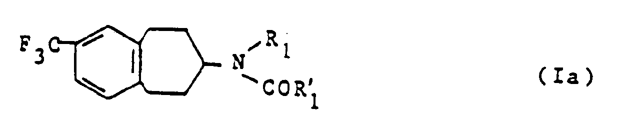 dans laquelle R₁ et R′₁ ont les significations précédemment définies,
lequel dérivé (Ia) peut être :
soit réduit pour donner le dérivé de formule générale Ib: 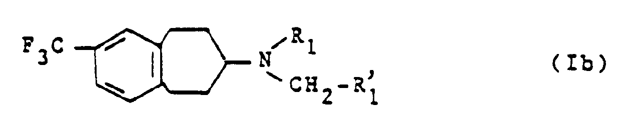 dans laquelle R₁ et R′₁ sont tels que précédemment définis,
soit hydrolysé pour donner le dérivé de formule générale Ic : 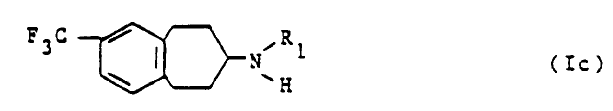 dans laquelle R₁ a la signification précédemment définie;
b) soit avec le chlorhydrate d'hydroxylamine pour obtenir l'oxime de formule : 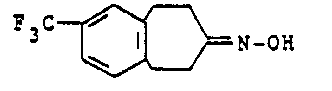 que l'on hydrogène sous pression , en présence d'un catalyseur comme le nickel de Raney, en milieu éthanol ammoniacal, pour obtenir le dérivé de formule Id : 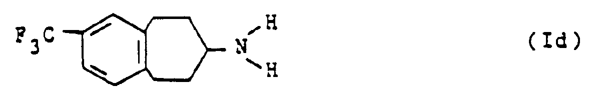 Ce dérivé (Id) peut être, à son tour, transformé selon les méthodes classiques de N-substitution, pour donner les amines secondaires et tertiaires correspondantes incluses dans la formule générale I;
c) soit avec le dérivé de formule IV
R₁-NH₂ (IV)
dans laquelle R₁ a la signification précédemment définie, en milieu acide chlorhydrique en présence d'un cyanure alcalin,
pour obtenir le dérivé de formule : 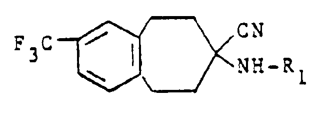 lequel est transformé en dérivé de formule : 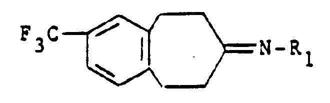 qui est, à son tour, transformé en dérivé de formule (Ie) 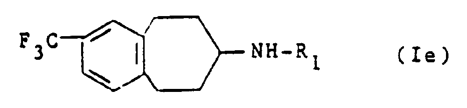 R₁ ayant, dans les trois dernières formules, la signification antérieurement définie;
d) soit avec un dérivé de formule V :
R₄-Hal (V)
dans laquelle R₄ représente un radical alcoyle ayant de 1 à 5 atomes de carbone en chaîne droite ou ramifiée, en présence de magnésium pour obtenir le dérivé de formule 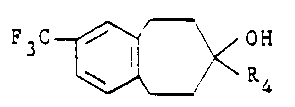 (dans laquelle R₄ a la signification précédemment définie) qui traité par un dérivé de formule R₅-CN (dans laquelle R₅ représente un radical alcoyle ayant de 1 à 4 atomes de carbone en chaîne droite ou ramifiée) en milieu sulfurique, donne le dérivé de formule : 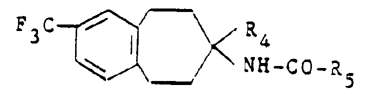 (dans laquelle R₄ et R₅ ont les significations précédemment définies)
lequel est réduit en un dérivé de formule If : 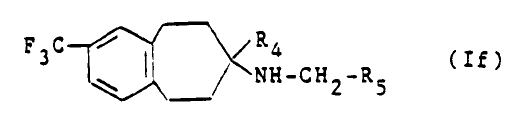 dans laquelle R₄ et R₅ ont les significations précédemment définies;
e) soit avec un dérivé de formule VI:
MCN (VI)
dans laquelle M représente un métal alcalin, en présence de CH₃CO OH et (CH₃CO)₂O,
pour donner le dérivé de formule : 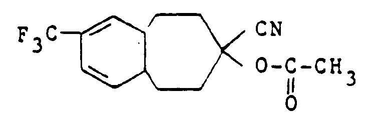 lequel est à son tour :
soit traité par Li AlH₄ pour donner le dérivé de formule Ig : 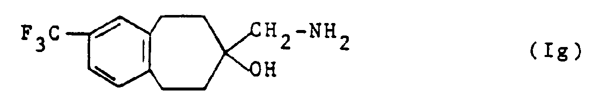 soit traité par une base en milieu aqueux pour donner le dérivé de formule : 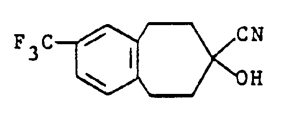 qui après déshydratation donne le dérivé de formule : 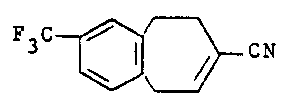 lequel par réduction donne le dérivé de formule Ih : 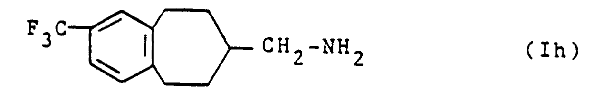
f) soit avec un dérivé de formule VII :
PO(OC₂H₅)₂-CH₂-CN (VII)
en présence d'une base pour donner le dérivé de formule : 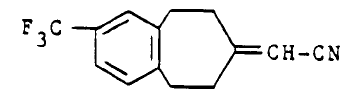 lequel par réduction donne le dérivé de formule Ii :

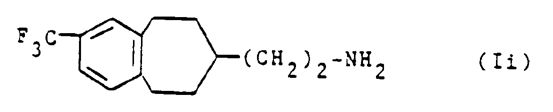

12. A titre de produit chimique nouveau utilisé comme matière première pour préparer les dérivés de la revendication 1, la trifluorométhyl-2 tétrahydro-6,7,8,9 [5H] benzocycloheptène-one-7.

13. Le procédé de préparation de la trifluorométhyl-2 tétrahydro-6,7,8,9 [5H] benzoxycloheptène-one-7, caractérisé en ce que :
- l'on nitre le p. trifluorométhyl iodobenzène pour obtenir le nitro-2 -trifluorométhyl-4 iodobenzène,
- l'on réduit ce dernier en amino-2 trifluorométhyl-4 iodobenzène,
- lequel est transformé, au moyen d'iodure de potassium, en trifluorométhyl-4 di-iodo-1,2 benzène.
- l'on fait réagir ce dernier avec l'acrylate de méthyle en présence de tri-o. tolyl phosphine, d'acétate de palladium, de triéthylamine, en chauffant dans un solvant adéquat, pour obtenir le trifluorométhyl-4 di-(β-méthoxycarbonylvinyl)-1,2 benzène ;
- que l'on hydrogène sous pression, en présence de catalyseur dans un solvant adéquat, pour obtenir le trifluorométhyl-4 di-(β-méthoxycarbonyléthyl)-1,2 benzène ;
- lequel est traité à reflux par une suspension d'hydrure de sodium dans un solvant adéquat pour donner un mélange de trifluorométhyl-2 méthoxycarbonyl-6 tétrahydro-6,7,8,9 [5H] benzocycloheptène-one 7 et de trifluorométhyl-2 méthoxycarbonyl-8 tétrahydro-6,7,8,9 [5H] benzocycloheptène-one 7 ;
- et l'on traite ce mélange ainsi obtenu par la soude en milieu hydro alcoolique pour obtenir la trifluorométhyl-2 tétrahydro-6,7,8,9 benzocycloheptene-one-7.

14. Les compositions pharmaceutiques contenant comme principe actif un dérivé selon les revendications 1, 2 et 4 à 10, avec des excipients pharmaceutiques appropriés.

## Revendications (Revendications pour l'(les) Etat(s) contractant(s) suivant(s): ES, GR)

1. Le procédé de préparation des dérivés du benzocycloheptène de formule générale I : 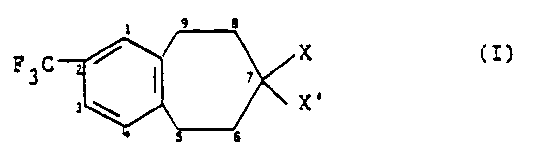 dans laquelle :
1- X représente un atome d'hydrogène, un radical alcoyle ayant de 1 à 5 atomes de carbone en chaîne droite ou ramifiée, un radical cyano ou un radical hydroxy,
2- X représente :
a) un radical hydroxy,
b) un radical OR
dans lequel R représente :
α)un radical acyle -COR′ dans lequel R′ représente
un radical alcoyle ayant de 1 à 5 atomes de carbone en chaîne droite ou ramifiée éventuellement substitué par un radical phényle, ou
un radical phényle éventuellement substitué par un ou plusieurs atomes d'halogène ou radicaux alcoyle ou alcoxy ayant chacun de 1 à 5 atomes de carbone ; ou
β)un radical alcoyle ayant de 1 à 5 atomes de carbone en chaîne droite ou ramifiée éventuellement substitué par un radical carboxy ou un radical phényle.
c) un radical de formule : 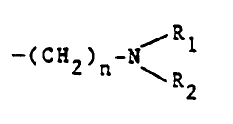
dans laquelle :
n représente 0, 1 ou 2, et
R₁ et R₂, identiques ou différents, représentent chacun :
un atome d'hydrogène ;
un radical alcoyle ayant de 1 à 5 atomes de carbone en chaîne droite ou ramifiée éventuellement substitué par un radical hydroxy, amino, carboxy, alcoxycarbonyle ayant de 1 à 6 atomes de carbone ou acyloxy de formule R′ CO-O- dans laquelle R′ a la signification précédemment définie ;
un radical alcényle ayant de 2 à 5 atomes de carbone ;
un radical alcynyle ayant de 2 à 5 atomes de carbone ;
un radical acyle de formule R˝CO dans laquelle R˝ représente un radical alcoyle ayant de 1 à 5 atomes de carbone en chaîne droite ou ramifiée,un radical phényle ou un radical phénylsulfonylamino dont la partie phényle est éventuellement substituée par un ou plusieurs atomes d'halogéne ou radicaux trifluorométhyle, alcoyle ou alcoxy ayant chacun de 1 à 5 atomes de carbone,
ou,
R₁ et R₂ représentent ensemble avec l'atome d'azote auquel ils sont liés :
- soit un groupe de formule 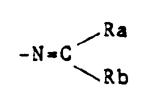 dans laquelle :
Ra représente un atome d'hydrogène ou un radical alcoyle ayant de 1 à 5 atomes de carbone en chaîne droite ou ramifiée, et
Rb représente un atome d'hydrogène, un radical alcoyle ayant de 1 à 5 atomes de carbone en chaîne droite ou ramifiée, un radical phényle ou un radical phénylalcoyle;
- soit un radical hétérocyclique penta- ou hexagonal renfermant éventuellement un autre hétéroatome : oxygène ou azote ;
ou
3- X et X′ représentent ensemble un radical de formule :
=N-R₃
dans laquelle
R₃ représente un radical alcoyle ayant de 1 à 5 atomes de carbone en chaîne droite ou ramifiée éventuellement substitué par
un radical cyano,
un reste - COOR‴ dans lequel R‴ représente un atome d'hydrogène ou un radical alcoyle ayant de 1 à 5 atomes de carbone ; ou
un radical phényle, lui-même éventuellement substitué par un ou plusieurs atomes d'halogène ou radicaux alcoyle ou alcoxy ayant chacun de 1 à 5 atomes de carbone ;
sous forme racémique et les énantiomères correspondants;
caractérisé en ce que l'on fait réagir :
la trifluorométhyl-2 tétrahydro-6,7,8,9 [5H] benzocycloheptène-one-7 de formule II : 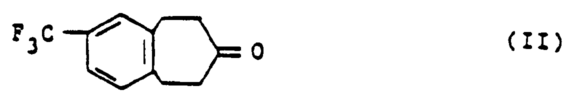
a) soit avec un dérivé carbonylé de formule générale III :
R′₁-CO-NH-R₁ (III)
dans laquelle R₁ a la signification énoncée précédemment et R′₁ représente :
un atome d'hydrogène ;
un radical alcoyle ayant de 1 à 4 atomes de carbone en chaîne droite ou ramifiée, en présence d'acide formique, pour obtenir un dérivé de formule générale Ia : 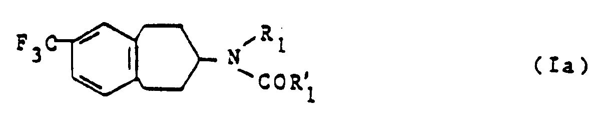 dans laquelle R₁ et R′₁ ont les significations précédemment définies,
lequel dérivé (Ia) peut être :
soit réduit pour donner le dérivé de formule générale Ib: 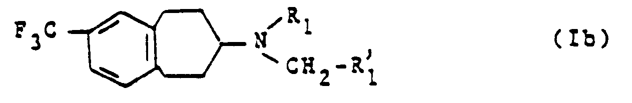 dans laquelle R₁ et R′₁ sont tels que précédemment définis,
soit hydrolysé pour donner le dérivé de formule générale Ic : 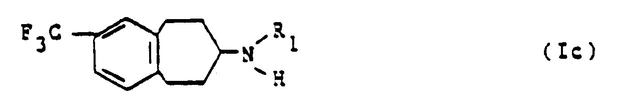 dans laquelle R₁ a la signification précédemment définie;
b) soit avec le chlorhydrate d'hydroxylamine pour obtenir l'oxime de formule : 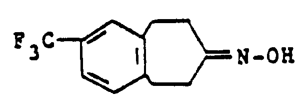 que l'on hydrogène sous pression , en présence d'un catalyseur comme le nickel de Raney, en milieu éthanol ammoniacal, pour obtenir le dérivé de formule Id : 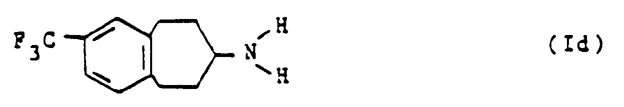 Ce dérivé (Id) peut être, à son tour, transformé selon les méthodes classiques de N-substitution, pour donner les amines secondaires et tertiaires correspondantes incluses dans la formule générale I;
c) soit avec le dérivé de formule IV
R₁-NH₂ (IV)
dans laquelle R₁ a la signification précédemment définie, en milieu acide chlorhydrique en présence d'un cyanure alcalin,
pour obtenir le dérivé de formule : 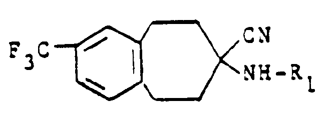 lequel est transformé en dérivé de formule : 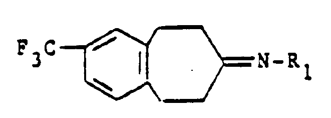 qui est, à son tour, transformé en dérivé de formule (Ie) 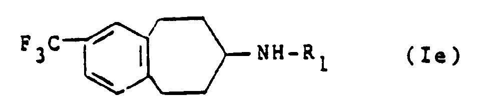 R₁ ayant, dans les trois dernières formules, la signification antérieurement définie;
d) soit avec un dérivé de formule V :
R₄-Hal (V)
dans laquelle R₄ représente un radical alcoyle ayant de 1 à 5 atomes de carbone en chaîne droite ou ramifiée, en présence de magnésium pour obtenir le dérivé de formule 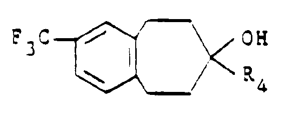 (dans laquelle R₄ a la signification précédemment définie) qui traité par un dérivé de formule R₅-CN (dans laquelle R₅ représente un radical alcoyle ayant de 1 à 4 atomes de carbone en chaîne droite ou ramifiée) en milieu sulfurique, donne le dérivé de formule : 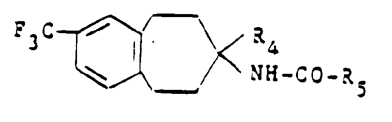 (dans laquelle R₄ et R₅ ont les significations précédemment définies)
lequel est réduit en un dérivé de formule If : 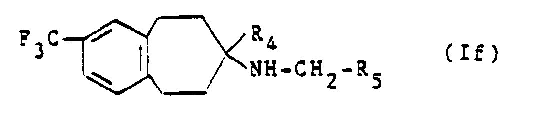 dans laquelle R₄ et R₅ ont les significations précédemment définies;
e) soit avec un dérivé de formule VI:
MCN (VI)
dans laquelle M représente un métal alcalin, en présence de CH₃CO OH et (CH₃CO)₂O, pour donner le dérivé de formule : 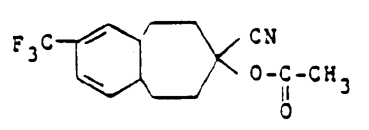 lequel est à son tour :
soit traité par Li AlH₄ pour donner le dérivé de formule Ig : 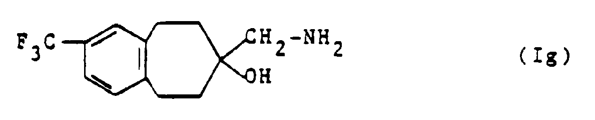 soit traité par une base en milieu aqueux pour donner le dérivé de formule : 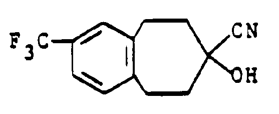 qui après déshydratation donne le dérivé de formule : 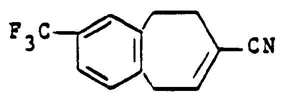 lequel par réduction donne le dérivé de formule Ih : 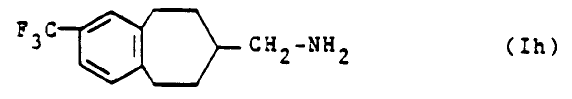
f) soit avec un dérivé de formule VII :
PO(OC₂H₅)₂-CH₂-CN (VII)
en présence d'une base pour donner le dérivé de formule ; 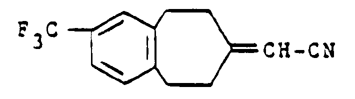 lequel par réduction donne le dérivé de formule Ii : 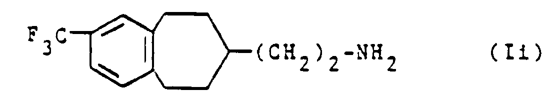 et, si on le désire, les dérivés Iₐ à Iᵢ précédemment obtenus qui renferment un groupe basique, sont traités avec des acides appropriés pour donner les sels d'addition acides correspondants.

2. Le procédé de préparation de la trifluorométhyl-2 tétrahydro-6,7,8,9 [5H] benzoxycloheptène-one-7, caractérisé en ce que :
- l'on nitre le p. trifluorométhyl iodobenzène pour obtenir le nitro-2 -trifluorométhyl-4 iodobenzène,
- l'on réduit ce dernier en amino-2 trifluorométhyl-4 iodobenzène,
- lequel est transformé, au moyen d'iodure de potassium, en trifluorométhyl-4 di-iodo-1,2 benzène.
- l'on fait réagir ce dernier avec l'acrylate de méthyle en présence de tri-o. tolyl phosphine, d'acétate de palladium, de triéthylamine, en chauffant dans un solvant adéquat, pour obtenir le trifluorométhyl-4 di-(β-méthoxycarbonylvinyl)-1,2 benzène ;
- que l'on hydrogène sous pression, en présence de catalyseur dans un solvant adéquat, pour obtenir le trifluorométhyl-4 di-(β-méthoxycarbonyléthyl)-1,2 benzène ;
- lequel est traité à reflux par une suspension d'hydrure de sodium dans un solvant adéquat pour donner un mélange de trifluorométhyl-2 méthoxycarbonyl-6 tétrahydro-6,7,8,9 [5H] benzocycloheptène-one 7 et de trifluorométhyl-2 méthoxycarbonyl-8 tétrahydro-6,7,8,9 [5H] benzocycloheptène-one 7 ;
- et l'on traite ce mélange ainsi obtenu par la soude en milieu hydro alcoolique pour obtenir la trifluorométhyl-2 tétrahydro-6,7,8,9 [5H] benzocycloheptène-one-7.

## Patentansprüche (Patentansprüche für folgende(n) Vertragsstaat(en): DE, GB, FR, IT, NL, SE, LI, CH, BE, AT, LU)

1. Benzocycloheptenderivat der allgemeinen Formel (I) 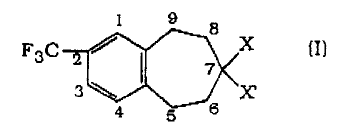 in der
1- X ein wasserstoffatom, eine geradkettige oder verzweigte Alkylgruppe mit 1 bis 5 Kohlenstoffatomen, eine Cyanogruppe oder eine Hydroxylgruppe bedeutet,
2- X′
a) eine Hydroxylgruppe
b) eine Gruppe OR
in der R
α) eine Acylgruppe-COR′, worin R′ für eine geradkettige oder verzweigte, gegebenenfalls durch eine Phenylgruppe substituierte Alkylgruppe mit 1 bis 5 Kohlenstoffatomen, oder
eine gegebenenfalls durch eines oder mehrere Halogenatome oder Alkyl- oder Alkoxygruppen mit jeweils 1 bis 5 Kohlenstoffatomen substituierte Phenylgruppe steht; oder
β) eine geradkettige oder verzweigte und gegebenenfalls durch eine Carboxygruppe oder eine Phenylgruppe substituierte Alkylgruppe mit 1 bis 5 Kohlenstoffatomen darstellt,
c) eine Gruppe der Formel 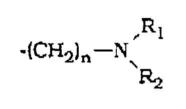
in der
n 0, 1 oder 2 und
R₁ und R₂, die gleich oder verschieden sein können, jeweils ein Wasserstoffatom;
eine geradkettige oder verzweigte, gegebenenfalls durch eine Hydroxylgruppe, Aminogruppe, Carboxygruppe, Alkoxycarbonylgruppe mit 1 bis 6 Kohlenstoffatomen oder Acyloxygruppe der Formel R′CO-O-, worin R′ die oben angegebenen Bedeutungen besitzt, substituierte Alkylgruppe mit 1 bis 5 Kohlenstoffatomen;
eine Alkenylgruppe mit 2 bis 5 Kohlenstoffatomen;
eine Alkinylgruppe mit 2 bis 5 Kohlenstoffatomen;
eine Acylgruppe der Formel R˝CO, worin R˝ eine geradkettige oder verzweigte Alkylgruppe mit 1 bis 5 Kohlenstoffatomen, eine Phenylgruppe oder eine Phenysulfonylaminogruppe darstellt, deren Phenylrest gegebenenfalls durch eines oder mehrere Halogenatome oder Trifluormethylgruppen, Alkyl- oder Alkoxygruppen mit jeweils 1 bis 5 Kohlenstoffatomen substituiert ist, darstellen, oder
R₁ und R₂ gemeinsam mit dem Stickstoffatom, an das sie gebunden sind,
- entweder eine Gruppe der Formel 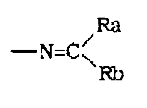 in der
Ra ein Wasserstoffatom oder eine geradkettige oder verzweigte Alkylgruppe mit 1 bis 5 Kohlenstoffatomen und
Rb ein Wasserstoffatom, eine geradkettige oder verzweigte Alkylgruppe mit 1 bis 5 Kohlenstoffatomen, eine Phenylgruppe oder eine Phenylalkylgruppe darstellen;
- oder eine 5-gliedrige oder 6-gliedrige heterocyclische Gruppe, die gegebenfalls ein weiteres Heteroatom, nämlich Sauerstoff oder Schwefel enthält, darstellen, bedeutet; oder
3- X und X′ gemeinsam eine Gruppe der Formel
= N-R₃
in der
R₃ eine geradkettige oder verzweigte, gegebenenfalls durch
eine Cyanogruppe,
einen Rest-COOR‴, worin R‴ ein Wasserstoffatom oder eine Alkylgruppe mit 1 bis 5 Kohlenstoffatomen darstellt; oder
eine Phenylgruppe, die ihrerseits gegebenenfalls durch eines oder mehrere Halogenatome oder Alkyl- oder Alkoxy-Gruppen mit jeweils 1 bis 5 Kohlenstoffatomen substituiert ist, substituierte Alkylgruppe mit 1 bis 5 Kohlenstoffatomen darstellt, bedeuten;
in Form des Racemats und der entsprechenden Enantiomeren.

2. Derivate nach Anspruch 1 der allgemeinen Formel (I′) 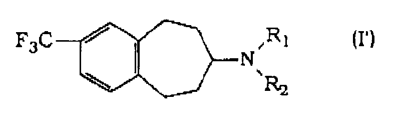 in der R₁ und R₂ die in Anspruch 1 angegebenen Bedeutungen besitzen.

3. Die Salze der eine basische Gruppe aufweisenden Derivate nach Anspruch 1 mit geeigneten Säuren.

4. Die Salze nach Anspruch 1, die physiologisch verträglich sind.

5. 2-Trifluormethyl-7-amino-[5H]-6,7,8,9-tetrahydro-benzocyclohepten.

6. 2-Trifluormethyl-7-(N-ethyl-N-formylamino)-[5H]-6,7,8,9-benzocyclohepten.

7. 2-Trifluormethyl-7-N-ethylamino-[5H]-6,7,8,9-tetrahydro-benzocyclohepten.

8. 2-Trifluormethyl-7-(N-ethyl-N-methylamino)-[5H]-6,7,8,9-tetrahydro-benzocyclohepten.

9. 2-Trifluormethyl-7-(N-ethoxycarbonylmethylamino)-[5H]-6,7,8,9-tetrahydro-benzocyclohepten.

10. 2-Trifluormethyl-7-(N-carboxymethylamino)-[5H]-6,7,8,9-tetrahydro-benzocyclohepten.

11. Verfahren zur Herstellung der Derivate nach Anspruch 1, **dadurch gekennzeichnet**, daß man 2-Trifluormethyl-[5H]-6,7,8,9-tetrahydro-benzocyclohepten-7-on der Formel (II) 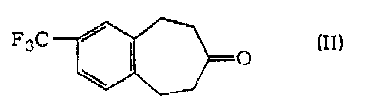
a) entweder mit einem Carbonylderivat der allgemeinen Formel (III)
R′₁-CO-NH-R₁ (III)
in der R₁ die oben angegebenen Bedeutungen besitzt und R′₁
ein Wasserstoffatom oder eine geradkettige oder verzweigte Alkylgruppe mit 1 bis 4 Kohlenstoffatomen bedeutet, in Gegenwart von Ameisensäure umsetzt zur Bildung eines Derivats der allgemeinen Formel (Ia) 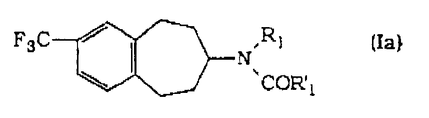 in der R₁ und R′₁ die oben angegebenen Bedeutungen besitzen, welches Derivat (Ia) entweder
reduziert werden kann zur Bildung des Derivats der allgemeinen Formel (Ib) 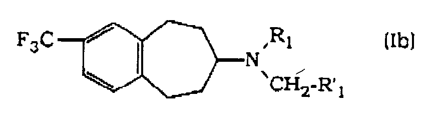 in der R₁ und R′₁ die oben angegebenen Bedeutungen besitzen, oder hydrolysiert werden kann zur Bildung des Derivats der allgemeinen Formel (Ic) 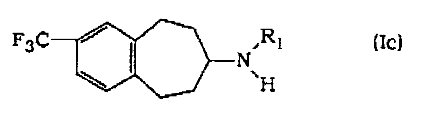 in der R₁ die oben angegebenen Bedeutungen besitzt;
b) oder mit Hydroxylamin-hydrochlorid umsetzt zur Bildung des Oxims der Formel 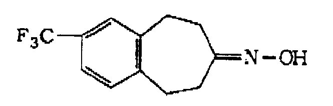 welches man unter Druck in Gegenwart eines Katalysators, wie Raney-Nickel, in ammoniakalischem Ethanol hydriert zur Bildung des Derivats der Formel (Id) 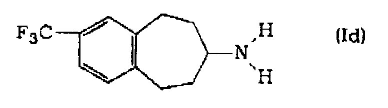 welches Derivat (Id) seinerseits unter Anwendung an sich bekannter Methoden der N-Substitution zu den entsprechenden sekundären und tertiären Aminen, die von der allgemeinen Formel (I) umfaßt sind, umgewandelt werden kann;
c) oder mit dem Derivat der Formel (IV)
R₁- NH₂
in der R₁ die oben angegebenen Bedeutungen besitzt, in chlorwasserstoffsaurem Medium in Gegenwart eines Alkalicyanids umsetzt zur Bildung des Derivats der Formel 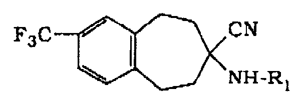 welches in das Derivat der Formel 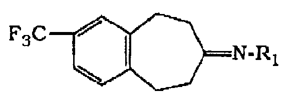 umgewandelt wird, das seinerseits in das Derivat der Formel (Ie) 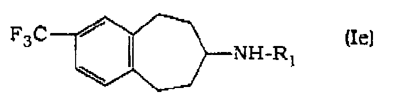 umgewandelt wird, in der R₁ in den drei zuletzt genannten Formeln die oben angegebenen Bedeutungen besitzt;
d) oder mit einem Derivat der Formel (V)
R₄-Hal (V)
in der R₄ eine geradkettige oder verzweigte Alkylgruppe mit 1 bis 5 Kohlenstoffatomen darstellt, in Gegenwart von Magnesium umsetzt zur Bildung des Derivats der Formel 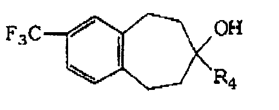 (worin R₄ die oben angegebenen Bedeutungen besitzt), welches durch Behandeln mit einem Derivat der Formel R₅-CN (worin R₅ eine geradkettige oder verzweigte Alkylgruppe mit 1 bis 4 Kohlenstoffatomen darstellt) in schwefelsaurem Medium das Derivat der Formel 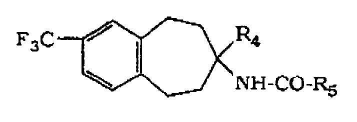 liefert, (worin R₄ und R₅ die oben angegebenen Bedeutungen besitzen),
welches zu einem Derivat der Formel (If) 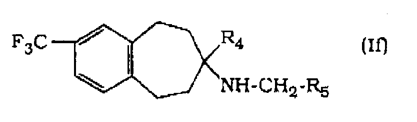 reduziert wird, worin R₄ und R₅ die oben angegebenen Bedeutungen besitzen;
e) oder mit einem Derivat der Formel (VI)
MCN (VI)
worin M ein Alkalimetall darstellt, in Gegenwart von CH₃COOH und (CH₃CO)₂O umsetzt,
zur Bildung des Derivats der Formel 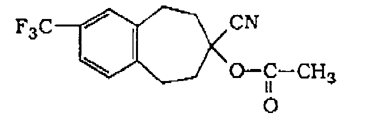 welches seinerseits
entweder mit Li AlH₄ behandelt wird zur Bildung des Derivats der Formel (Ig) 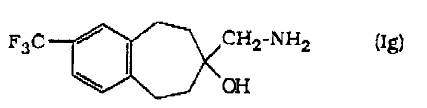 oder in wäßrigem Medium mit einer Base behandelt wird zur Bildung des Derivats der Formel 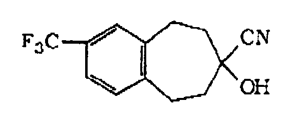 welches durch Dehydratisieren das Derivat der Formel 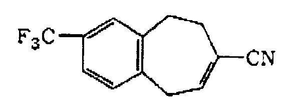 liefert, welches durch Reduktion das Derivat der Formel (Ih) 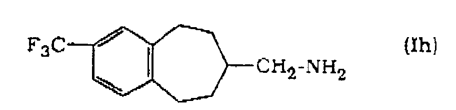 ergibt;
f) oder mit einem Derivat der Formel (VII):
PO(OC₂H₅)₂-CH₂-CN (VII)
in Gegenwart einer Base umsetzt zur Bildung des Derivats der Formel 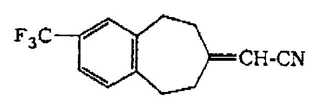 welches durch Reduktion das Derivat der Formel (Ii) 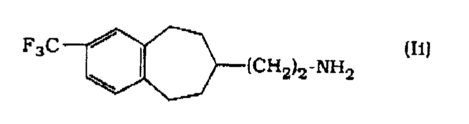 ergibt.

12. Zwischenprodukt zur Herstellung der Derivate nach Anspruch 1, nämlich 2-Trifluormethyl-[5H]-6,7,8,9-tetrahydro-benzocyclohepten-7- on.

13. Verfahren zur Herstellung von 2-Trifluormethyl-[5H]-6,7,8,9-tetrahydro-benzocyclohepten-7-on, **dadurch gekennzeichnet**, daß man
- p-Trifluormethyl-iodbenzol zu 2-Nitro-4-trifluormethyl-iodbenzol nitriert,
- welches man zu 2-Amino-4-trifluormethyl-iodbenzol reduziert,
- welches man mit Hilfe von Kaliumiodid in 4-Trifluormethyl-1,2-diiodbenzol umwandelt,
- welches man mit Methylacrylat in Gegenwart von Tri-o-tolylphosphin, Palladiumacetat und Triethylamin durch Erhitzen in einem geeigneten Lösungsmittel zu 4-Trifluormethyl-1,2-di(β-methoxycarbonylvinyl)-benzol umsetzt;
- welches man unter Druck in Gegenwart eines Katalysators in einem geeigneten Lösungsmittel zu 4-Trifluormethyl-1,2-di(β-methoxycarbonylethyl)-benzol hydriert;
- welches man am Rückfluß mit einer Suspension von Natriumhydrid in einem geeigneten Lösungsmittel am Rückfluß behandelt zur Bildung einer Mischung aus 2-Trifluormethyl-6-methoxycarbonyl-[5H]-6,7,8,9-tetrahydro-benzocyclohepten-7-on und 2-Trifluormethyl-8-methoxycarbonyl-[5H]-6,7,8,9-tetrahydro-benzocyclohepten-7-on;
- und die in dieser Weise erhaltene Mischung in wäßrig alkoholischem Medium mit Natriumhydroxid behandelt zur Bildung von 2-Trifluormethyl-[5H]-6,7,8,9-tetrahydro-benzocyclohepten-7-on.

14. Pharmazeutische Zubereitung enthaltend als Wirkstoff ein Derivat nach einem der Ansprüche 1, 2 und 4 bis 10 zusammen mit pharmazeutisch geeigneten Trägermaterialien.

## Patentansprüche (Patentansprüche für folgende(n) Vertragsstaat(en): ES, GR)

1. Verfahren zur Herstellung von Benzocycloheptenderivaten der allgemeinen Formel (I) 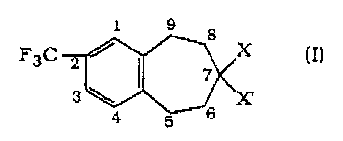 in der
1- X ein Wasserstoffatom, eine geradkettige oder verzweigte Alkylgruppe mit 1 bis 5 Kohlenstoffatomen, eine Cyanogruppe oder eine Hydroxylgruppe bedeutet,
2- X′
a) eine Hydroxylgruppe
b) eine Gruppe OR
in der R
α) eine Acylgruppe - COR′, worin R′ für eine geradkettige oder verzweigte, gegebenenfalls durch eine Phenylgruppe substituierte Alkylgruppe mit 1 bis 5 Kohlenstoffatomen, oder
eine gegebenenfalls durch eines oder mehrere Halogenatome oder Alkyl- oder Alkoxygruppen mit jeweils 1 bis 5 Kohlenstoffatomen substituierte Phenylgruppe steht; oder
β) eine geradkettige oder verzweigte und gegebenenfalls durch eine Carboxygruppe oder eine Phenylgruppe substituierte Alkylgruppe mit 1 bis 5 Kohlenstoffatomen darstellt,
c) eine Gruppe der Formel 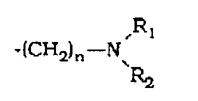
in der
n 0, 1 oder 2 und
R₁ und R₂, die gleich oder verschieden sein können, jeweils ein Wasserstoffatom;
eine geradkettige oder verzweigte, gegebenenfalls durch eine Hydroxylgruppe, Aminogruppe, Carboxygruppe, Alkoxycarbonylgruppe mit 1 bis 6 Kohlenstoffatomen oder Acyloxygruppe der Formel R′CO-O-, worin R′ die oben angegebenen Bedeutungen besitzt, substituierte Alkylgruppe mit 1 bis 5 Kohlenstoffatomen;
eine Alkenylgruppe mit 2 bis 5 Kohlenstoffatomen;
eine Alkinylgruppe mit 2 bis 5 Kohlenstoffatomen;
eine Acylgruppe der Formel R˝CO, worin R˝ eine geradkettige oder verzweigte Alkylgruppe mit 1 bis 5 Kohlenstoffatomen, eine Phenylgruppe oder eine Phenysulfonylaminogruppe darstellt, deren Phenylrest gegebenenfalls durch eines oder mehrere Halogenatome oder Trifluormethylgruppen, Alkyl- oder Alkoxygruppen mit jeweils 1 bis 5 Kohlenstoffatomen substituiert ist, darstellen, oder
R₁ und R₂ gemeinsam mit dem Stickstoffatom, an das sie gebunden sind,
- entweder eine Gruppe der Formel 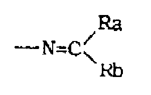 in der
Ra ein Wasserstoffatom oder eine geradkettige oder verzweigte Alkylgruppe mit 1 bis 5 Kohlenstoffatomen und
Rb ein Wasserstoffatomen, eine geradkettige oder verzweigte Alkylgruppe mit 1 bis 5 Kohlenstoffatomen, eine Phenylgruppe oder eine Phenylalkylgruppe darstellen;
- oder eine 5-gliedrige oder 6-gliedrige heterocyclische Gruppe, die gegebenfalls ein weiteres Heteroatom, nämlich Sauerstoff oder Schwefel enthält, darstellen, bedeutet; oder
3- X und X′ gemeinsam eine Gruppe der Formel
= N-R₃
in der
R₃ eine geradkettige oder verzweigte, gegebenenfalls durch
eine Cyanogruppe,
einen Rest -COOR‴, worin R‴ ein Wasserstoffatom oder eine Alkylgruppe mit 1 bis 5 Kohlenstoffatomen darstellt; oder
eine Phenylgruppe, die ihrerseits gegebenenfalls durch eines oder mehrere Halogenatome oder Alkyl- oder Alkoxy-Gruppen mit jeweils 1 bis 5 Kohlenstoffatomen substituiert ist, substituierte Alkylgruppe mit 1 bis 5 Kohlenstoffatomen darstellt, bedeuten;
in Form des Racemats und der entsprechenden Enantiomeren,
**dadurch gekennzeichnet**, daß man 2-Trifluormethyl-[5H]-6,7,8,9-tetrahydrobenzocyclohepten-7-on der Formel (II) 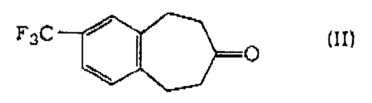
a) entweder mit einem Carbonylderivat der allgemeinen Formel (III)
R′₁-CO-NH-R₁ (III)
in der R₁ die oben angegebenen Bedeutungen besitzt und R′₁
ein Wasserstoffatom oder eine geradkettige oder verzweigte Alkylgruppe mit 1 bis 4 Kohlenstoffatomen bedeutet, in Gegenwart von Ameisensäure umsetzt zur Bildung eines Derivats der allgemeinen Formel (Ia) 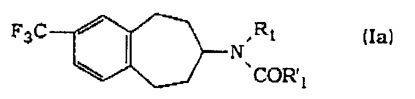 in der R₁ und R′₁ die oben angegebenen Bedeutungen besitzen, welches Derivat (Ia) entweder
reduziert werden kann zur Bildung des Derivats der allgemeinen Formel (Ib) 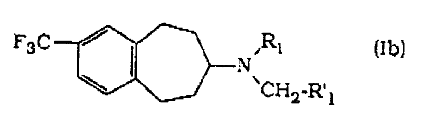 in der R₁ und R′₁ die oben angegebenen Bedeutungen besitzen,
oder hydrolysiert werden kann zur Bildung des Derivats der allgemeinen Formel (Ic) 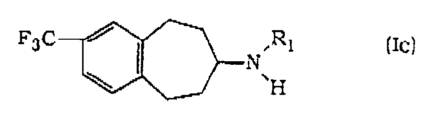 in der R₁ die oben angegebenen Bedeutungen besitzt;
b) oder mit Hydroxylamin-hydrochlorid umsetzt zur Bildung des Oxims der Formel 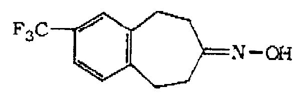 welches man unter Druck in Gegenwart eines Katalysators, wie Raney-Nickel, in ammoniakalischem Ethanol hydriert zur Bildung des Derivats der Formel (Id) 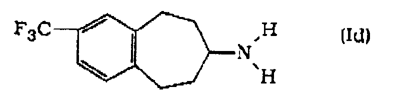 welches Derivat (Id) seinerseits unter Anwendung an sich bekannter Methoden der N-Substitution zu den entsprechenden sekundären und tertiären Aminen, die von der allgemeinen Formel (I) umfaßt sind, umgewandelt werden kann;
c) oder mit dem Derivat der Formel (IV)
R₁-NH₂
in der R₁ die oben angegebenen Bedeutungen besitzt, in chlorwasserstoffsaurem Medium in Gegenwart eines Alkalicyanids umsetzt zur Bildung des Derivats der Formel 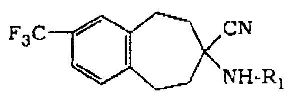 welches in das Derivat der Formel 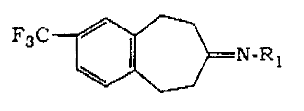 umgewandelt wird,
das seinerseits in das Derivat der Formel (Ie) 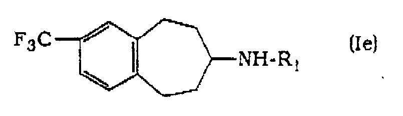 umgewandelt wird, in der R₁ in den drei zuletzt genannten Formeln die oben angegebenen Bedeutungen besitzt;
d) oder mit einem Derivat der Formel (V)
R₁-Hal (V)
in der R₄ eine geradkettige oder verzweigte Alkylgruppe mit 1 bis 5 Kohlenstoffatomen darstellt, in Gegenwart von Magnesium umsetzt zur Bildung des Derivats der Formel 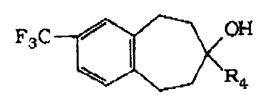 (worin R₄ die oben angegebenen Bedeutungen besitzt), welches durch Behandeln mit einem Derivat der Formel R₅-CN (worin R₅ eine geradkettige oder verzweigte Alkylgruppe mit 1 bis 4 Kohlenstoffatomen dar stellt) in schwefelsaurem Medium das Derivat der Formel 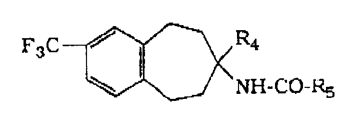 liefert, (worin R₄ und R₅ die oben angegebenen Bedeutungen besitzen),
welches zu einem Derivat der Formel (If) 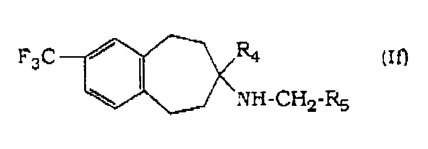 reduziert wird, worin R₄ und R₅ die oben angegebenen Bedeutungen besitzen;
e) oder mit einem Derivat der Formel (VI)
MCN (VI)
worin M ein Alkalimetall darstellt, in Gegenwart von CH₃COOH und (CH₃CO)₂O umsetzt,
zur Bildung des Derivats der Formel 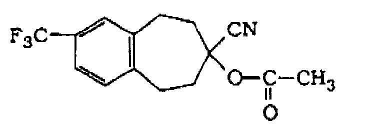 welches seinerseits
entweder mit Li AlH₄ behandelt wird zur Bildung des Derivats der Formel (Ig) 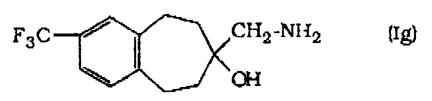 oder in wäßrigem Medium mit einer Base behandelt wird zur Bildung des Derivats der Formel 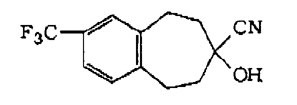 welches durch Dehydratisieren das Derivat der Formel 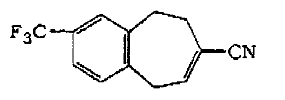 liefert, welches durch Reduktion das Derivat der Formel (Ih) 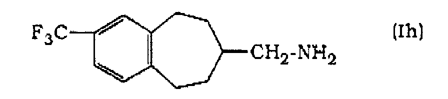 ergibt;
f) oder mit einem Derivat der Formel (VII):
PO(OC₂H₅)₂-CH₂-CN (VII)
in Gegenwart einer Base umsetzt zur Bildung des Derivats der Formel 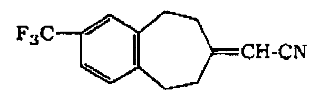 welches sich durch Reduktion des Derivats der Formel (Ii) 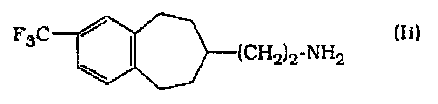 ergibt und gewünschtenfalls die in der obigen Weise erhaltenen Derivate (Ia) bis (Ii), die eine basische Gruppe enthalten, mit geeigneten Säuren behandelt zur Bildung der entsprechenden Säureadditionssalze.

2. Verfahren zur Herstellung von 2-Trifluormethyl-[5H]-6,7,8,9-tetrahydro-benzocyclohepten-7-on, **dadurch gekennzeichnet**, daß man
- p-Trifluormethyl-iodbenzol zu 2-Nitro-4-trifluormethyl-iodbenzol nitriert,
- welches man zu 2-Amino-4-trifluormethyl-iodbenzol reduziert.
- welches man mit Hilfe von Kaliumiodid in 4-Trifluormethyl-1,2-diiodbenzol umwandelt,
- welches man mit Methylacrylat in Gegenwart von Tri-o-tolylphosphin, Palladiumacetat und Triethylamin durch Erhitzen in einem geeigneten Lösungsmittel zu 4-Trifluormethyl-1,2-di-(β-methoxycarbonylvinyl)-benzol umsetzt;
- welches man unter Druck in Gegenwart eines Katalysators in einem geeigneten Lösungsmittel zu 4-Trifluormethyl-1,2-di-(β-methoxycarbonylethyl)-benzol hydriert;
- welches man am Rückfluß mit einer Suspension von Natriumhydrid in einem geeigneten Lösungsmittel am Rückfluß behandelt zur Bildung einer Mischung aus 2-Trifluormethyl-6-methoxycarbonyl-[5H]-6,7,8,9-tetrahydro-benzocyclohepten-7-on und 2-Trifluormethyl-8-methoxycarbonyl-[5H]-6,7,8,9-tetrahydro-benzocyclohepten-7-on;
- und die in dieser Weise erhaltene Mischung zur wäßrig alkoholischem Medium mit Natriumhydroxid behandelt zur Bildung von 2-Trifluormethyl-[5H]-6,7,8,9-tetrahydro-benzocyclohepten-7-on.

## Claims (Claims for the following Contracting State(s): DE, GB, FR, IT, NL, SE, LI, CH, BE, AT, LU)

1. Benzocycloheptene compounds of the general formula I : 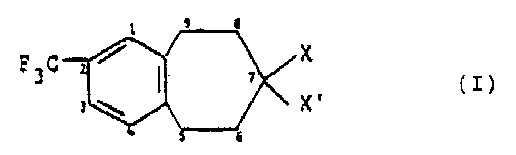 wherein :
1- X represents a hydrogen atom, an alkyl radical having from 1 to 5 carbon atoms in straight or branched chain, a cyano radical or a hydroxy radical,
2- X′ represents :
a) a hydroxy radical,
b) an OR radical wherein R represents:
α) an acyl radical -COR′ wherein R′ represents an alkyl radical having from 1 to 5 carbon atoms in straight or branched chain which is optionally substituted by a phenyl radical, or represents
a phenyl radical which is optionally substituted by one or more halogen atoms or by one or more alkyl or alkoxy radicals each having from 1 to 5 carbon atoms;
or
β) an alkyl radical having from 1 to 5 carbon atoms in straight or branched chain which is optionally substituted by a carboxy radical or by a phenyl radical,
c) a radical of formula : 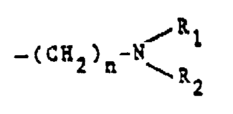
wherein
n represents 0, 1 or 2, and
R₁ and R₂, which are the same or different, each represents :
a hydrogen atom;
an alkyl radical having from 1 to 5 carbon atoms in straight or branched chain which is optionally substituted by a hydroxy, amino or carboxy radical, by an alkoxycarbonyl radical having from 1 to 6 carbon atoms or by an acyloxy radical of the formula R′CO-O- wherein R′ is as defined hereinbefore;
an alkenyl radical having from 2 to 5 carbon atoms;
an alkynyl radical having from 2 to 5 carbon atoms;
an acyl radical of formula R˝CO wherein R˝ represents an alkyl radical having from 1 to 5 carbon atoms in straight or branched chain, a phenyl radical or a phenylsulphonylamino radical of which the phenyl moiety is optionally substituted by one or more halogen atoms or trifluoromethyl radicals, or by one or more alkyl or alkoxy radicals each having from 1 to 5 carbon atoms, or
R₁ and R₂, together with the nitrogen atom to which they are bonded, represent :
- either a group of formula 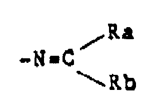 wherein :
Ra represents a hydrogen atom or an alkyl radical having from 1 to 5 carbon atoms in straight or branched chain, and
Rb represents a hydrogen atom, an alkyl radical having from 1 to 5 carbon atoms in straight or branched chain, a phenyl radical or a phenyl-alkyl radical;
- or a pentagonal or hexagonal heterocyclic radical optionally containing an additional hetero atom : oxygen or nitrogen;
or
3- X and X′ together represent a radical of formula :
=N-R₃
wherein
R₃ represents an alkyl radical having from 1 to 5 carbon atoms in straight or branched chain which is optionally substituted by
a cyano radical,
a -COOR‴ radical wherein R‴ represents a hydrogen atom or an alkyl radical having from 1 to 5 carbon atoms; or
a phenyl radical, itself optionally substituted by one or more halogen atoms or by one or more alkyl or alkoxy radicals each having from 1 to 5 carbon atoms;
in racemic form or in the form of corresponding enantiomers.

2. The compounds of claim 1 corresponding to the general formula I′ : 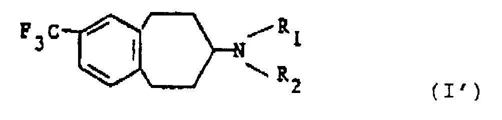 wherein R₁ and R₂ are as defined in claim 1.

3. The salts of the compounds of claim 1 having a basic group with appropriate acids.

4. The salts according to claim 3 that are physiologically tolerable.

5. 2-Trifluoromethyl-7-amino-6,7,8,9-tetrahydro[5H]benzocycloheptene.

6. 2-Trifluoromethyl-7-(N-ethyl-N-formylamino)-6,7,8,9-tetrahydro[5H]benzocycloheptene.

7. 2-Trifluoromethyl-7-N-ethylamino-6,7,8,9-tetrahydro[5H]benzocycloheptene.

8. 2-Trifluoromethyl-7-(N-ethyl-N-methylamino)-6,7,8,9-tetrahydro[5H]benzocycloheptene.

9. 2-Trifluoromethyl-7-(N-ethoxycarbonylmethylamino)-6,7,8,9-tetrahydro[5H]benzocycloheptene.

10. 2-Trifluoromethyl-7-(N-carboxymethylamino)-6,7,8,9-tetrahydro[5H]benzocycloheptene.

11. A process for the preparation of the compounds of claim 1, characterised in that
2-trifluoromethyl-6,7,8,9-tetrahydro[5H]benzocyclohepten-7-one of formula II : 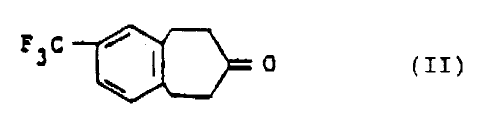 is reacted
a) with a carbonyl compound of the general formula III :
R′₁-CO-NH-R₁ (III)
wherein R₁ is as defined hereinbefore and R′₁ represents:
a hydrogen atom, or
an alkyl radical having from 1 to 4 carbon atoms in straight or branched chain,
in the presence of formic acid, to yield a compound of the general formula Ia : 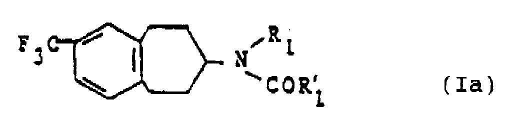 wherein R₁ and R′₁ are as defined hereinbefore, which compound (Ia) may be :
either reduced to yield the compound of the general formula Ib: 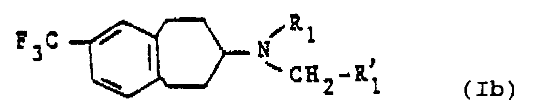 wherein R₁ and R′₁ are as defined hereinbefore, or hydrolysed to yield the compound of the general formula Ic : 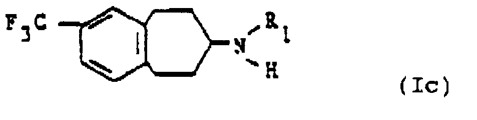 wherein R₁ is as defined hereinbefore;
b) with hydroxylamine hydrochloride to yield the oxime of formula : 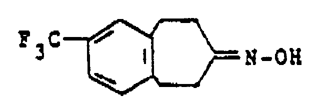 which is hydrogenated under pressure, in the presence of a catalyst such as Raney nickel, in an ammoniacal ethanol medium, to yield the compound of formula Id : 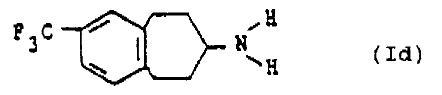 which compound (Id) may, in turn, be converted according to conventional methods of N-substitution to yield the corresponding secondary and tertiary amines included in the general formula I;
c) with the compound of formula IV
R₁-NH₂ (IV)
wherein R₁ is as defined hereinbefore, in hydrochloric acid medium in the presence of an alkali metal cyanide, to yield the compound of formula : 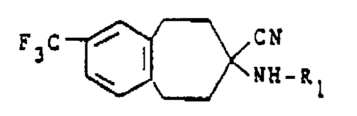 which is converted into the compound of formula : 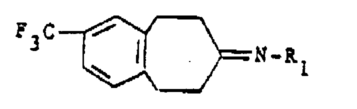 which, in turn, is converted into the compound of formula (Ie) 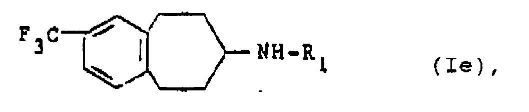 R₁ in the last three formulae being as defined hereinbefore;
d) with a compound of formula V :
R₄-Hal (V)
wherein R₄ represents an alkyl radical having from 1 to 5 carbon atoms in straight or branched chain, in the presence of magnesium to yield the compound of formula 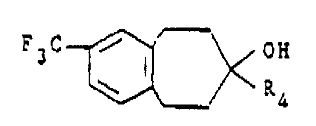 (wherein R₄ is as defined hereinbefore) which, treated with a compound of formula R₅-CN (wherein R₅ represents an alkyl radical having from 1 to 4 carbon atoms in straight or branched chain) in sulphuric medium, yields the compound of formula : 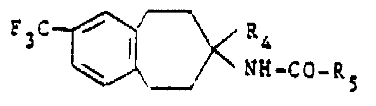 (wherein R₄ and R₅ are as defined hereinbefore) which is reduced to a compound of formula If : 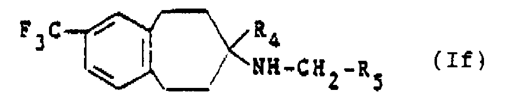 wherein R₄ and R₅ are as defined hereinbefore;
e) with a compound of formula VI:
MCN (VI)
wherein M represents an alkali metal, in the presence of CH₃COOH and (CH₃CO)₂O,
to yield the compound of formula : 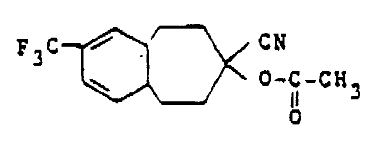 which, in turn, is
either treated with LiAlH₄ to yield the compound of formula Ig : 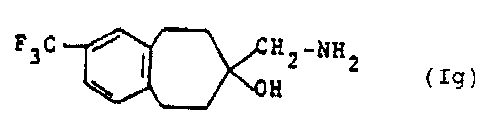 or treated with a base in aqueous medium to yield the compound of formula : 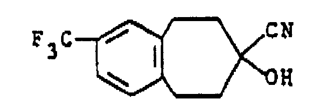 which, after dehydration, yields the compound of formula : 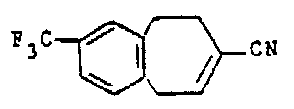 which on reduction yields the compound of formula Ih : 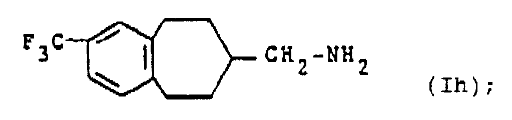
f) or with a compound of formula VII :
PO(OC₂H₅)₂-CH₂-CN (VII)
in the presence of a base to yield the compound of formula : 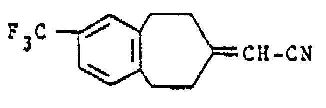 which on reduction yields the compound of formula Ii :

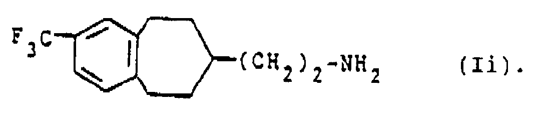

12. 2-Trifluoromethyl-6,7,8,9-tetrahydro[5H]benzocyclohepten-7-one as a new chemical product used as the starting material to prepare the compounds of claim 1.

13. A process for the preparation of 2-trifluoromethyl-6,7,8,9-tetrahydro[5H]benzocyclohepten-7-one, characterised in that :
- p-trifluoromethyliodobenzene is nitrated to yield 2-nitro-4-trifluoromethyliodobenzene,
- the latter is reduced to 2-amino-4-trifluoromethyliodobenzene,
- which is converted, by means of potassium iodide, into 4-trifluoromethyl-1,2-di-iodobenzene,
- the latter is reacted with methyl acrylate in the presence of tri-o.-tolylphosphine, palladium acetate and triethylamine, with heating in a suitable solvent, to yield 4-trifluoromethyl-1,2-di-(β-methoxycarbonylvinyl)benzene;
- which is hydrogenated under pressure, in the presence of a catalyst in a suitable solvent, to yield 4-trifluoromethyl-1,2-di-(β-methoxycarbonylethyl)-benzene;
- which is treated under reflux with a suspension of sodium hydride in a suitable solvent to yield a mixture of 2-trifluoromethyl-6-methoxycarbonyl-6,7,8,9-tetrahydro[5H]benzocyclohepten-7-one and 2-trifluoromethyl-8-methoxycarbonyl-6,7,8,9-tetrahydro[5H]benzocyclohepten-7-one;
- and the mixture so obtained is treated with sodium hydroxide in aqueous alcoholic medium to yield 2-trifluoromethyl-6,7,8,9-tetrahydrobenzocyclohepten-7-one.

14. Pharmaceutical compositions comprising as active ingredient a compound according to claims 1, 2 and 4 to 10 together with suitable pharmaceutical excipients.

## Claims (Claims for the following Contracting State(s): ES, GR)

1. A process for the preparation of benzocycloheptene compounds of the general formula I : 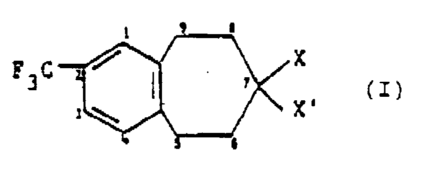 wherein :
1- X represents a hydrogen atom, an alkyl radical having from 1 to 5 carbon atoms in straight or branched chain, a cyano radical or a hydroxy radical,
2- X′ represents :
a) a hydroxy radical,
b) an OR radical wherein R represents:
α) an acyl radical -COR′ wherein R′ represents an alkyl radical having from 1 to 5 carbon atoms in straight or branched chain which is optionally substituted by a phenyl radical, or represents
a phenyl radical which is optionally substituted by one or more halogen atoms or by one or more alkyl or alkoxy radicals each having from 1 to 5 carbon atoms;
or
β) an alkyl radical having from 1 to 5 carbon atoms in straight or branched chain which is optionally substituted by a carboxy radical or by a phenyl radical,
c) a radical of formula : 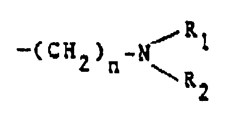
wherein
n represents 0, 1 or 2, and
R₁ and R₂, which are the same or different, each represents :
a hydrogen atom;
an alkyl radical having from 1 to 5 carbon atoms in straight or branched chain which is optionally substituted by a hydroxy, amino or carboxy radical, by an alkoxycarbonyl radical having from 1 to 6 carbon atoms or by an acyloxy radical of the formula R′CO-O- wherein R′ is as defined hereinbefore;
an alkenyl radical having from 2 to 5 carbon atoms;
an alkynyl radical having from 2 to 5 carbon atoms;
an acyl radical of formula R˝CO wherein R˝ represents an alkyl radical having from 1 to 5 carbon atoms in straight or branched chain, a phenyl radical or a phenylsulphonylamino radical of which the phenyl moiety is optionally substituted by one or more halogen atoms or trifluoromethyl radicals, or by one or more alkyl or alkoxy radicals each having from 1 to 5 carbon atoms,
or
R₁ and R₂, together with the nitrogen atom to which they are bonded, represent :
- either a group of formula 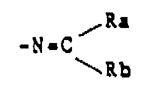 wherein :
Ra represents a hydrogen atom or an alkyl radical having from 1 to 5 carbon atoms in straight or branched chain, and
Rb represents a hydrogen atom, an alkyl radical having from 1 to 5 carbon atoms in straight or branched chain, a phenyl radical or a phenyl-alkyl radical;
- or a pentagonal or hexagonal heterocyclic radical optionally containing an additional hetero atom : oxygen or nitrogen;
or
3- X and X′ together represent a radical of formula :
=N-R₃
wherein
R₃ represents an alkyl radical having from 1 to 5 carbon atoms in straight or branched chain which is optionally substituted by
a cyano radical,
a -COOR‴ radical wherein R‴ represents a hydrogen atom or an alkyl radical having from 1 to 5 carbon atoms; or
a phenyl radical, itself optionally substituted by one or more halogen atoms or by one or more alkyl or alkoxy radicals each having from 1 to 5 carbon atoms;
in racemic form or in the form of corresponding enantiomers, characterised in that 2-trifluoromethyl-6,7,8,9-tetrahydro[5H]benzocyclohepten-7-one of formula II : 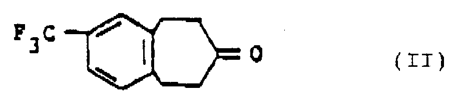 is reacted
a) with a carbonyl compound of the general formula III :
R′₁-CO-NH-R₁ (III)
wherein R₁ is as defined hereinbefore and R′₁ represents:
a hydrogen atom, or
an alkyl radical having from 1 to 4 carbon atoms in straight or branched chain,
in the presence of formic acid, to yield a compound of the general formula Ia : 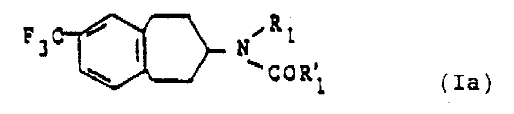 wherein R₁ and R′₁ are as defined hereinbefore, which compound (Ia) may be :
either reduced to yield the compound of the general formula Ib: 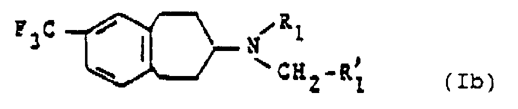 wherein R₁ and R′₁ are as defined hereinbefore, or hydrolysed to yield the compound of the general formula Ic : 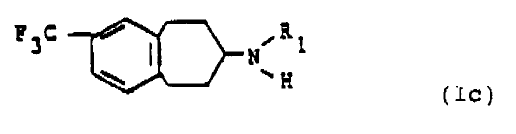 wherein R₁ is as defined hereinbefore;
b) with hydroxylamine hydrochloride to yield the oxime of formula : 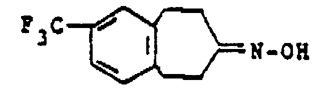 which is hydrogenated under pressure, in the presence of a catalyst such as Raney nickel, in an ammoniacal ethanol medium, to yield the compound of formula Id : 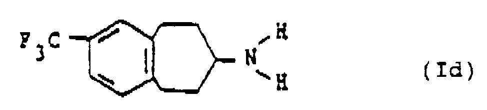 which compound (Id) may, in turn, be converted according to conventional methods of N-substitution to yield the corresponding secondary and tertiary amines included in the general formula I;
c) with the compound of formula IV
R₁-NH₂ (IV)
wherein R₁ is as defined hereinbefore, in hydrochloric acid medium in the presence of an alkali metal cyanide, to yield the compound of formula : 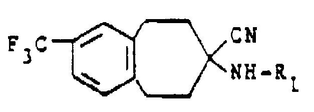 which is converted into the compound of formula : 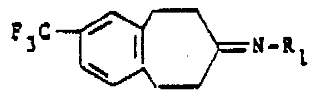 which, in turn, is converted into the compound of formula (Ie) 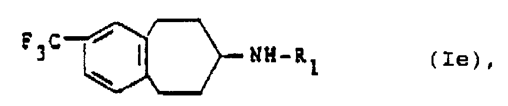 R₁ in the last three formulae being as defined hereinbefore;
d) with a compound of formula V :
R₄-Hal (V)
wherein R₄ represents an alkyl radical having from 1 to 5 carbon atoms in straight or branched chain, in the presence of magnesium to yield the compound of formula 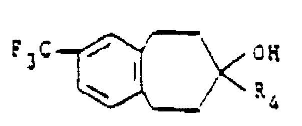 (wherein R₄ is as defined hereinbefore) which, treated with a compound of formula R₅-CN (wherein R₅ represents an alkyl radical having from 1 to 4 carbon atoms in straight or branched chain) in sulphuric medium, yields the compound of formula : 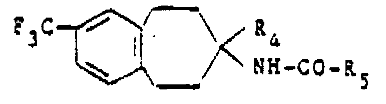 (wherein R₄ and R₅ are as defined hereinbefore) which is reduced to a compound of formula If : 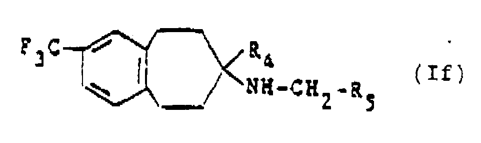 wherein R₄ and R₅ are as defined hereinbefore;
e) with a compound of formula VI:
MCN (VI)
wherein M represents an alkali metal, in the presence of CH₃COOH and (CH₃CO)₂O,
to yield the compound of formula : 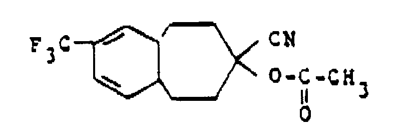 which, in turn, is
either treated with LiAlH₄ to yield the compound of formula Ig : 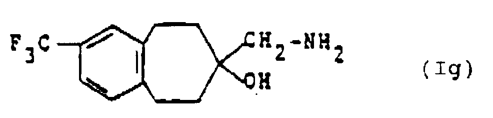 or treated with a base in aqueous medium to yield the compound of formula : 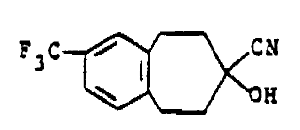 which, after dehydration, yields the compound of formula : 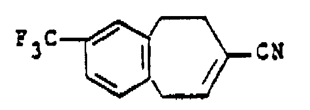 which on reduction yields the compound of formula Ih : 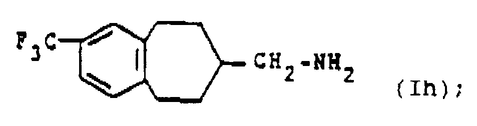
f) or with a compound of formula VII :
PO(OC₂H₅)₂-CH₂-CN (VII)
in the presence of a base to yield the compound of formula : 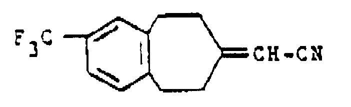 which on reduction yields the compound of formula Ii : 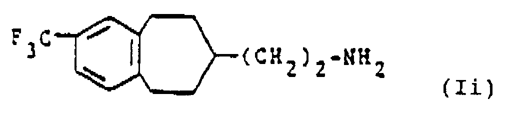 and, if desired, the compounds Ia to Ii obtained above that contain a basic group are treated with appropriate acids to yield the corresponding acid addition salts.

2. A process for the preparation of 2-trifluoromethyl-6,7,8,9-tetrahydro[5H]benzocyclohepten-7-one, characterised in that :
- p-trifluoromethyliodobenzene is nitrated to yield 2-nitro-4-trifluoromethyliodobenzene,
- the latter is reduced to 2-amino-4-trifluoromethyliodobenzene,
- which is converted, by means of potassium iodide, into 4-trifluoromethyl-1,2-di-iodobenzene,
- the latter is reacted with methyl acrylate in the presence of tri-o.-tolylphosphine, palladium acetate and triethylamine, with heating in a suitable solvent, to yield 4-trifluoromethyl-1,2-di-(β-methoxycarbonylvinyl)benzene;
- which is hydrogenated under pressure, in the presence of a catalyst in a suitable solvent, to yield 4-trifluoromethyl-1,2-di-(β-methoxycarbonylethyl)-benzene;
- which is treated under reflux with a suspension of sodium hydride in a suitable solvent to yield a mixture of 2-trifluoromethyl-6-methoxycarbonyl-6,7,8,9-tetrahydro[5H]benzocyclohepten-7-one and 2-trifluoromethyl-8-methoxycarbonyl-6,7,8,9-tetrahydro[5H]benzocyclohepten-7-one;
- and the mixture so obtained is treated with sodium hydroxide in aqueous alcoholic medium to yield 2-trifluoromethyl-6,7,8,9-tetrahydro[5H]benzocyclohepten-7-one.
